# EUROPEAN PATENT SPECIFICATION

(11) **EP 3 691 738 B1**
(45) Date of publication and mention of the grant of the patent: **29.04.2026**
(21) Application number: 18864754.9
(22) Date of filing: 05.10.2018
(51) Int. Cl.: A61K 35/28, A61L 29/04, A61L 29/08, A61L 29/12, A61L 29/14, A61L 29/16, A61L 31/06, A61L 31/10, A61L 31/12, A61L 31/14, A61F 2/24, A61F 2/02, A61K 35/545, A61L 31/04, A61P 9/00

(54) **IMPLANTABLE BIOREACTOR**
IMPLANTIERBARER BIOREAKTOR
BIORÉACTEUR IMPLANTABLE

(30) Priority: 05.10.2017 US 201762568348 P
(43) Date of publication of application: 12.08.2020
(73) Proprietor: The Johns Hopkins University, Baltimore, MD 21218 (US)
(72) Inventor: HWANG, Chao-Wei, West Friendship, Maryland 21794 (US); JOHNSTON, Peter, Baltimore, MD 21212 (US); GERSTENBLITH, Gary, Reisterstown, Maryland 21136 (US); WEISS, Robert G., Hunt Valley, Maryland 21030 (US); TOMASELLI, Gordon, Lutherville, Maryland 21093 (US); SCHULMAN, Steven, Reisterstown, Maryland 21136 (US)
(74) Representative: Murgitroyd & Company
(86) International application number: PCT/US2018/054623
(87) International publication number: WO 2019/071135

(56) References cited:
- WO-A2-2006/080009
- WO-A2-2009/061382
- AU-B2- 2013 257 540
- US-A1- 2001 044 413
- US-A1- 2006 136 049
- US-A1- 2012 083 767
- US-A1- 2015 209 299
- US-A1- 2015 217 030
- US-A1- 2017 245 976
- US-A1- 2017 245 976
- US-A1- 2018 098 867
- US-B2- 9 456 893
- US-B2- 9 788 978
- ANONYMOUS: "ATCC Stem Cell Culture Guide", ATCC AMERICAN TYPE CULTURE COLLECTION, 1 January 2015 (2015-01-01), XP055588517, Retrieved from the Internet <URL:https://www.atcc.org/~/media/7E031EF950594BC3B85A411AE1DC9684.ashx> [retrieved on 20190514]
- ANONYMOUS: "ATCC Stem Cell Culture Guide", ATCC AMERICAN TYPE CULTURE COLLECTION, 2015, pages 1, XP055588517

## Description

### BACKGROUND OF THE INVENTION

Stem cells, and the products they produce, hold the promise to regenerate damaged tissue and improve healing following injury. Such therapy may limit adverse remodeling after a tissue injury, such as myocardial infarction (MI). Adverse remodeling, i.e. an increase in left ventricular volume as assessed by change in end systolic volume (ESV) and end diastolic volume (EDV) after MI, is a potent predictor of mortality and the development of heart failure. The effects of stem cell therapy on adverse remodeling in many clinical trials, however, are modest to date. One reason may be that with many of the methods currently used to deliver stem cells to patients, whether by intravenous or intracoronary infusion, or by direct myocardial injection, it is likely that only a small percentage of the cells remain in the heart, and of those that do, few survive for any significant period of time. At the same time there is growing evidence from pre-clinical studies that many of the positive effects of stem cell therapy result from the release of growth factors, cytokines, chemokines, nucleic acids, exosomes, and other molecules and vesicles. These and other components of the cell secretome are collectively referred to as "paracrine factors." It follows that if cells delivered to the heart remain there and survive for only a limited period, the time they have to exert the beneficial effects via paracrine mechanisms is limited as well, which may be one explanation for the modest beneficial effects seen in most clinical trials.

Clinical trials using intra-coronary and intra-myocardial injection of stem cells in an attempt to promote healing and regeneration of infarcted myocardium have produced modest results to date. Potential reasons for the modest results include inadequate levels of paracrine factors, which may be due to poor retention of cells due to cell death, removal via immunologic mechanisms, and simple "washout" following delivery, leaving the cells with only a brief opportunity to exert beneficial effects.

As such, there still exists an unmet need for improved methods for delivering paracrine factors to organs that are in need of repair and healing.

US 2017/245976 A1 discloses an implantable bioreactor containing a barrier which is designed to allow the release of cell-derived biomolecules, but restricts the entry of immunologic and other cells, or the egress of the cells contained within the bioreactor. In an embodiment, the implantable bioreactor comprises a pouch; and cells enclosed within the pouch, said cells being capable of producing paracrine factors, wherein the pouch is collapsible and expandable to be intravascularly implantable, wherein the pouch is semipermeable such that it provides containment of the cells, preventing the egress of the cells while further providing a barrier that shields the cells from immunological attack, and wherein the pouch comprises multiple pores having a diameter of at least about 0.1 µm, such that the pores are capable of releasing out of the pouch paracrine factors that enhance recovery of injured tissue. WO 2006/080009 discloses an implantable bioreactor device comprising a first compartment configured to be capable of fluidic communication with a vasculature of a subject; and a second compartment configured for containing cells, said second compartment being separated from said first compartment by a membrane. The membrane may block passage of said cells from said second compartment to said first compartment. AU 2013257540 B2 discloses compositions for the biological repair of cartilage using a hybrid construct combining both an inert structure and living. The inert structure comprises concentric internal and external and inflatable/expandable balloon-like bio-polymers. The living core comprises the cell-matrix construct comprised of Human Dermal Fibroblasts, for example, seeded in a scaffold.

### SUMMARY OF THE INVENTION

To improve stem cell survival and retention, and thereby increase the time stem cells can exert beneficial paracrine-mediated effects, the present inventors have developed an implantable bioreactor. The bioreactor is an enclosure which houses a population of cells including, for example, stem cells and in some embodiments, includes other non-stem cell types, having a semi-permeable membrane that allows for free exchange of paracrine factors, nutrients and wastes, but not of cells, and is related to the inventors' first generation bioreactor disclosed in U.S. Patent Publication No. 2012/0083767 A1, filed October 3, 2011. By preventing bioreactor cell escape and host immune cell entry, the bioreactor provides a protected environment within which the contained cells survive for an extended period *in vivo* while releasing paracrine factors, thus allowing for an extended time to provide beneficial effects. The implantable bioreactor can be for systemic or local delivery of paracrine factors. The bioreactor can be optionally adhered to another medical device.

The present invention is set out in the appended set of claims.

### BRIEF DESCRIPTION OF THE DRAWINGS

Figure 1 illustrates the first generation (G1) Stem Cell Implantable Bioreactor (SCIB) for *in vitro* paracrine factor production and cell viability experiments.
Figure 2: Paracrine factor release from prototype G1-SCIBs in vitro. G1-SCIBs containing 10⁶ human MSCs released relevant PFs to surrounding media when cultured over 7 days.
Figures 3A-3C: Prototype G1-SCIB *in vivo.* A) Fluoroscopic image of the prototype G1-SCIB implanted via the right internal jugular vein. B) Xenogeneic human MSCs continue to release relevant PFs from the prototype SCIB following one week *in vivo* in the pig, and C) showed normal MSC morphology and growth characteristics in culture.
Figure 4: G1-SCIB therapy reduced adverse remodeling 4 weeks post-MI as measured by a reduction of the amount of increase in ESV (*p*=0.036 cells vs. placebo) and EDV (*p*=0.059 cells vs. placebo).
Figure 5: Scar size as measured by late gadolinium-enhanced MRI 3 days and 4 weeks after MI. While scar size changed in both the cell and placebo group, SCIB-based cell therapy conferred an increase in heterogeneous gray scar coupled with a decrease in dense core scar.
Figure 6: Scanning electron micrographs of track-etched (poly)ethylene terephthalate (PET) film show a dense pattern of uniform cylindrical pores (low magnification LEFT; high magnification RIGHT).
Figure 7A A schematic drawing of one embodiment of the G2 SCIB apparatus showing a dual lumen pouch on the catheter with the following dimensions: 0.064" catheter shaft 30 cm shaft length, 2 mm tapered catheter tip, Single 0.014" OTW guidewire port, Single cell infusion port, Luer-compatible connections, and twin radio-opaque markers.
Figure 7B A photograph depicting an embodiment of the pouch of the bioreactor. The phot shows G2-SCIB cell chamber design with the following specifications: Polyethylene terephthalate constructions, about 25-um wall thickness, about 6.0 mm diameter, about 120 mm length, having track-etched pores of about 2 µm in diameter, about 7-8 x 10⁶ pores / cm². The chamber or pouch can comprise non-cellular support structures such as inner microbeads (customizable), and/or an inner hydrogel (customizable).
Figure 8 depicts a series of photograph of a dual lumen pouch or chamber of the present invention with photomicrographs detailing the track-etched pores of about 2 µm in diameter, and their high, uniform density, and at two different magnifications.

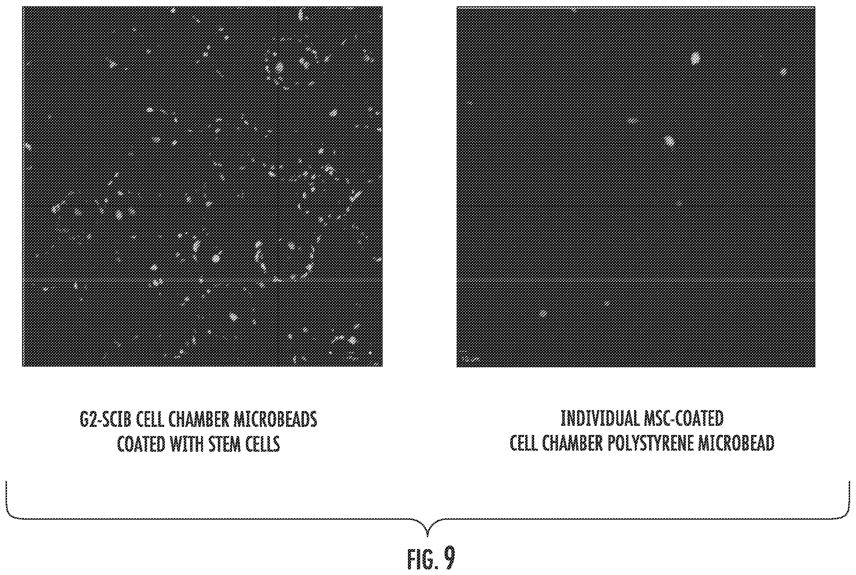

Figure 9 shows that mesenchymal stem cells (MSCs) can adhere to fibronectin coated polystyrene beads, wherein the beads provide internal cell adhesion microsurfaces to increase cell capacity and viability within the bioreactor cell pouch or chamber. Using green/red live/dead cell staining after 7 days in culture in the cell chamber, the photomicrographs show live MSCs adhering to the polystyrene beads and show a singular bead with live MSCs at a higher magnification. Greater than 90% cell viability within the cell chamber was achieved at 7 days, and the live cells were distributed throughout the chamber or pouch.
Figures 10A-10B: Permeability across track-etched PET membrane pouch with 0.44 µm diameter pores. (A) FITC labeled bovine serum albumin is released rapidly from the SCIB; (B) Exosomes (labeled red) also freely cross the membrane and are readily incorporated by H9c2 rat cardiomyoblasts (green).
Figures 11A-11B: Bioluminescence imaging (BLI) of mouse MSCs in G2-SCIB pouches (A) is significantly higher when MSCs are loaded in the SCIB pouch with a hydrogel matrix (B, blue) than without (B, orange). Higher bioluminescence is correlated with higher cell viability.
Figures 12A-12B: A) VEGF release from G2-SCIB (orange) is in excess of 10-fold greater than that from G1-SCIB (blue). B) Consistent with this, endothelial tubulogenesis induced by G2-SCIB also exceeds that induced by G1-SCIB.
Figure 13: Example of an embodiment of the bioreactor enclosure of the present invention with permeable and semi-permeable membranes enclosing a cell culture matrix or nanofiber cloud.
Figure 14: Example of an embodiment of the rolled enclosure bioreactor with alternating layers having nanofiber mesh and a cell layer.

### DETAILED DESCRIPTION OF THE INVENTION

Embodiments of the implantable bioreactor disclosed herein can solve the problems of diffusion or washout by providing adequate, prolonged delivery of paracrine factors secreted from the bioreactor while protecting the contents of the bioreactor from washout or immunologic clearance in an enclosed housing. The invention includes in one embodiment, a minimally invasive percutaneous bioreactor and, in another embodiment, an implantable device, both of which can adequately produce and release paracrine factors. The bioreactor can also be used to promote healing and regeneration in any other tissue or organ by release of paracrine factors.

As used herein, "bioreactor" refers to a collection of cells in a housing or enclosure, or sandwiched within a semi-permeable membrane which allows the release of paracrine factors generated by the cells within the pouch. The bioreactor can include multiple housings, enclosures, or lumens. The bioreactor can be placed *in situ* in a mammal with or without the use of a catheter. In some embodiments, the bioreactor can be placed *in situ* in the vascular space of a mammal with a catheter or similar apparatus.

As used herein, "paracrine factors" are diffusible components produced by one cell to affect another cell. The diffusible components can be any component of the cell secretome, including any protein, growth factor, nucleic acid, nuclear material, virus, viral vector (including vectors used in gene therapy), lipid, biomolecule, cytokine, chemokine, vesicle, exosome, nutrient or fluid produced by the cells housed in the bioreactor. The permeability characteristics of the inventive pouch is an important feature of the invention as it is designed to allow the release and movement of paracrine factors, nutrients, wastes, and signaling factors into and out of the enclosure, but does not allow the entry of immunologic cells or the entry or egress of stem cells or other types of cells. One of skill in the art, would understand that "paracrine factors" include many factors, including exosomes, which are vesicles typically 50 nm to 200 nm in diameter. Without being held to any particular theory, it is also contemplated that these factors are among the prime mediators of any protective benefits resulting from stem or other cell types that are introduced, *in vivo,* for the purpose of supporting tissue repair. Examples of paracrine factors include factors that promote angiogenesis (e.g. VEGF, HGF, ANG), cytoprotection (e.g. IGF-1, LIF, TMSB4), cell proliferation (e.g. FGF, PGF, SCG), cell migration (e.g. THBS1, SDF-1, PDGF), as well as secreted exosomes which incorporate one or more of these factors.

As used herein, the term "semi-permeable" means that in some embodiments, the outer membrane of the enclosure will allow molecules, proteins, peptides, nucleic acids, vesicles and the like of a certain size pass through the membrane, and objects of a larger size, such as cells, cannot pass through. These types of membranes are commercially available and have different molecular weight cut off values or pore sizes. In other embodiments, term "semi-permeable" is used to mean the presence of micropores created with of specific diameters in a membrane or surface which is not permeable, to allow proteins, peptides, nucleic acids, vesicles and the like of a certain size pass through the membrane, and objects of a larger size, such as cells, cannot pass through.

As used herein, "stem cells" can include, but are not limited to, embryonic, adult, and induced pluripotent stem cells. Embryonic stem cells include, without limitation, totipotent, pluripotent and multipotent stem cells, and adult stem cells include, without limitation, mesenchymal stem cells, cardiac stem cells, adipose-derived stem cells and endothelial stem cells. In some embodiments, any non-stem types of cells can also be included, such as, for example, fibroblasts, cardiomyocytes, endothelial cells, other paracrine factor secreting cells (such as, without limitation, hepatocytes, pancreatic islet cells, bone marrow cells), or engineered cells and any combinations thereof. In some embodiments, cell clusters, such as cardiospheres, and mixtures of different stem cells and non-stem cells can also be included. In some embodiments, the cells can include "modified" or "engineered" cells, such as, for example, genetically modified cells (*e.g.,* cells transfected with genes encoding growth factors which may or may not be native to the cell or cells), cells transformed from another cell type (*e.g.,* induced pluripotent stem cells and cells derived from these cells), or cells labeled with a detectable moiety (*e.g.,* cells with fluorescent tags or transfected cells with genes encoding for bioluminescence).

Various embodiments include (a) an implantable bioreactor which enhances recovery of injured myocardium and other tissue utilizing a cell strategy; (b) a percutaneously implantable bioreactor, which also includes an easily retrievable percutaneous bioreactor allowing removal once an intended treatment period is complete; (c) a temporary implantable device that releases paracrine factors, which are generated *de novo* by cells; (d) a permanent implantable device that releases paracrine factors, which are generated *de novo* by cells; (e) a bioreactor implanted via a standard vascular sheath; (f) an implantable bioreactor which locally releases paracrine factors in the target tissue; (g) an implantable bioreactor which contains a barrier with pores which allow the release of cell-derived biomolecules, but not large enough to allow the entry of immunologic and other cells, or the egress of the cells contained within the bioreactor; (h) an implantable bioreactor which contains a barrier composed of the materials described herein and which is designed to allow the release of cell-derived biomolecules, but not large enough to allow the entry of immunologic and other cells, or the egress of the cells contained within the bioreactor; (i) an implantable bioreactor which systemically releases paracrine factors; (j) an implantable bioreactor having multi-tube and multi-lumen cell chambers or pouches in a catheter; (k) and an implantable bioreactor having within the cell chamber(s) or pouch(es), non-cellular support structures, such as microbeads or nanofibers or hydrogel matrices.

In accordance with one or more embodiments, the bioreactors can be designed for either systemic delivery or local delivery of paracrine factors. The bioreactors encompass embodiments for implantation via any clinical technique, including, but not limited to, surgery, laparoscopic, percutaneous, endoscopic, arthroscopic, or bronchoscopic techniques. The bioreactors, in various embodiments, can adhere to a medical device.

In some embodiments, the systemic delivery implantable bioreactor comprises an enclosure housing stem cells or other cell types which produce and release paracrine factors. The cell enclosure comprises, in one embodiment, a physical enclosure fabricated with a semi-permeable membrane, or in another embodiment fabricated with a microporous polymer matrix encapsulating the stem cells. The cell enclosure includes micropores impermeable to cells, but of sufficient size to allow free permeation of fluid, paracrine factors, wastes and nutrients so they can be transferred efficiently and without hindrance. In some embodiments, the implantable bioreactor is deployed in the intravascular space (such as central veins and large arteries); implantation into any other body cavity, orifice, tissue, blood vessel, organ, or skin (such as in wounds) is also contemplated. In some embodiments, the implantable bioreactor is temporarily implanted and retrieved later. In other embodiments, the implantable bioreactor remains indefinitely as a permanent implant.

The implantable bioreactors can be designed for *systemic* or for *local* delivery of produced bio-products at the target. Both types encompass embodiments for implantation via any clinical technique, including, but not limited to, surgery, laparoscopic, percutaneous, endoscopic, arthroscopic, or bronchoscopic techniques. Both types, in various embodiments, can adhere to a medical device.

In one embodiment, the systemic delivery implantable bioreactor comprises an enclosure housing stem cells and/or other cell types which produce and release paracrine factors. The cell enclosure comprises, in one embodiment, a physical enclosure fabricated with a semi-porous membrane, and in another embodiment fabricated with a microporous polymer matrix encapsulating the stem cells. The cell enclosure includes micropores impermeable to cells, but of sufficient size to allow free permeation of fluid, paracrine factors, waste and nutrients so they can be transferred efficiently and without hindrance.

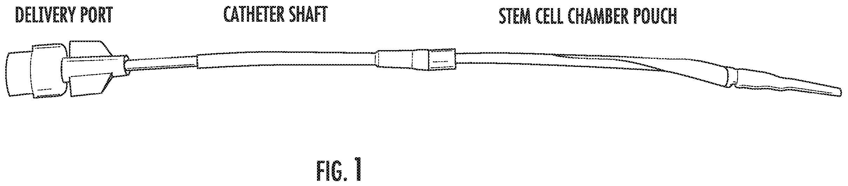

In another embodiment of a systemic delivery bioreactor, the enclosure of the implantable bioreactor is an enclosure which contains stem cells, other cell types, and/or media in its lumen. In various embodiments, the enclosure can be (a) stand-alone or (b) mounted on a wire or catheter or (c) mounted as part of another implantable device, in which case it can be implanted along with the other device. In any of these instances, the bioreactor can be implanted by any clinical technique, including, but not limited to, surgical implantation, percutaneous implantation, or insertion via any body orifice or wound, or placement via laparoscopic, endoscopic, arthroscopic or bronchoscopic techniques. In any of these instances, the bioreactor can be removed. In an embodiment, the enclosure can be prefilled with its intended contents or, if attached to a catheter, filled and potentially emptied and re-filled during and after implantation. When percutaneously implanted, the device can be passed directly or via a standard vascular sheath, for placement into the intravascular space, for example (possible embodiments are illustrated in Figures 1, 7 and 8).

The bioreactor comprises one or more cell chambers or pouches which serve as enclosures for cells. (a) Structure. In some embodiments, the cell chamber/pouch is attached to the body or the distal end of one or more catheter tubes and optionally spans across at least one open port to enable infusion, sampling, and/or circulation of materials into the cell pouch via the catheter. No restriction is placed on the geometric shape of the cell pouch; for example, it may be smooth and cylindrical, or shaped with multiple crevices and surface undulations. The maximal diameter of the cell pouch is such that it does not completely span and occlude the anatomic structure within which it is placed. To aid in pouch positioning, radio-opaque markers can be placed at either end of the pouch, within the pouch, or on the attached catheter.

In some embodiments, the bioreactor catheter can be closed, and not comprise an open port.

To summarize, there can be multiple variations of the embodiments of the bioreactor of the present invention. Such variations can comprise, for example, one or more catheter tubes; each catheter tube can comprise one or more cell chambers or pouches; each cell chambers or pouch can span one or more catheter tubes.

(b) Semi-permeability. The walls of the bioreactor enclosure cell pouch are semi-permeable. The wall is an immunoprotective barrier, and is therefore impermeable to cells, but permeable to the components of the cell secretome (including growth factors, proteins, lipids, exosomes, nuclear materials, and extracellular vesicles) as well as to wastes and nutrients. A specific range of pore sizes that satisfies these conditions is pore diameters from 50 nm to 5000 nm.

(c) Inner immunoprotective structures. In accordance with some embodiments, cells are placed in the bioreactor enclosure cell pouch either stand-alone or within immunoprotective structures. These immunoprotective structures are well described in the literature, and include hydrogel microspheres (e.g., based on chitosan, alginate, collagen, gelatin, agarose, and their various derivatives) and enclosures, cellulose-based enclosures, nanofiber-based matrices, or microfabricated cellular enclosures. Characteristics of these immunoprotective structures include semi-permeability; that is, they are impermeable to cells, yet permeable to nutrients, wastes, and components of the cell secretome.

(d) Material Composition. In accordance with some embodiments, the bioreactor enclosure cell chamber/pouch can be composed of one or a combination of a wide spectrum of biocompatible synthetic and non-synthetic materials. Examples of synthetic materials include polymers and co-polymers of polyesters and derivatives specifically including poly(ethylene terephthalate) and derivatives, fluoropolymers specifically including polytetrafluoroethylene and derivatives, polycarbonates and derivatives, poly(ethylene-co-vinyl acetate) and derivatives, poly(n-butyl methacrylate) and derivatives, poly(styrene-b-isobutylene-b-styrene) and derivatives, polycaprolactone and derivatives, polyimides and derivatives, polyurethanes and derivatives, polysulfones and derivatives, polyacrylonitrile and derivatives, polymethylmethacrylate and derivatives, poly(lactic acid), poly(lactide-co-glycolide), silicones. Non-polymeric materials could include silicon and derivatives. Examples of non-synthetic materials include cellulose-based materials and derivatives, collagen-based materials and derivatives, and extracellular matrix-based materials and derivatives. The materials can be naturally porous (such as cellulose or expanded polytetrafluoroethylene), or made porous by various manufacturing methods (such as track-etching, laser micro-drilling, micro-machining, photolithographic etching, chemical etching).

Coatings: The interior surface of the bioreactor enclosure can be coated with molecules which enhance cell attachment and function. Specific examples include fibronectin, polylysine, proteins with Arg-Gly-Asp sequences, and any derivative. The interior surface can also be surface-modified to enhance cell attachment and function. Specific examples of this process include corona-treatment and plasma-treatment. The exterior surface of the bioreactor enclosure can be coated or surface-modified to minimize thrombosis and/or fibrosis, reduce immunogenicity, improve biocompatibility, or other substances to improve sustained deliverability. The adjustment of coatings or surface modification is well known to those of ordinary skill in the art. Specific examples include attaching heparin chains or polyethylene glycol chains to the external surface or fluoropassivation treatment of the external surface. Additionally, drug delivery devices can be attached to the cell chamber/pouch to enable delivery of any drug or agent into the inner microenvironment or to the outside of the cell pouch. Such devices can include, for example, hydrogels, polymeric matrices, beads, and nanoparticles comprising drugs or biologically active agents.

Geometry: The physical shape of the bioreactor enclosure can be designed to be smooth or to incorporate surface undulations and crevices to maximize surface area for mass transfer.

Bioreactor contents: Any number of native or genetically altered cell types or stem cell lines can be used in the device based on the type of injury and organ that is being targeted for healing and/or regeneration. The cells can be used stand-alone or bathed in a media containing any number of proteins, growth factors, nucleic acids, or other molecules or vesicles. Specific and non-exhaustive examples of the bioreactor contents are outlined in the following paragraphs.

Cell Chamber/Pouch Inner Environment: The cell pouch inner microenvironment comprises at least one or more of the following components: (a) One or more types of paracrine factor producing cells; (b) Non-cellular support structures; (c) One or more types of supporting cells. The cell pouch inner microenvironment can also contain cell culture media. All of these components may be customized by the end-user at any time to control paracrine factor output, release kinetics, and paracrine factor type. Additionally, all components of the inner microenvironment can be modified to contain agents that enhance cell attachment and proliferation (e.g., polylysine, fibronectin, or proteins with Arg-Gly-Asp sequences), reduce thrombosis (e.g., heparin, direct thrombin inhibitors, or anti-platelet agents), reduce inflammation (e.g., with steroids), reduce infection (e.g., with antibiotics or antiviral agents), modulate cell growth (e.g., with growth factors), or to deliver any drug or agent into the inner microenvironment or to the outside of the cell pouch. Additionally, the inner microenvironment can contain drug delivery devices to enable delivery of any drug or agent into the inner microenvironment only or to the outside of the cell pouch as well. Such devices can include, for example, hydrogels, polymeric matrices, beads, and nanoparticles comprising drugs or biologically active agents.

In some embodiments the bioreactor is an enclosure, as described above, which is attached to catheter tubing with ports connecting the pouch lumen to the portion of the catheter exterior to the patient, allowing infusion, sampling, and circulation of cells and media. In another embodiment, multiple lumens within the housing of the catheter can provide additional options for continuous circulation of cells within the pouch, and an open distal port can be used as an intravenous line or for central venous pressure. Alternatively, the bioreactor can be mounted directly on a wire.

As used herein, the term "non-cellular support structures" can include, without limitation, physical things such as beads, microparticles, nanofiber, as well as a cell culture matrix. The cell culture matrix can include, for example, hydrogels, scaffolds, nanofibers, microparticles, and can be based on biologic, synthetic, or any combination of different biologic and/or synthetic materials. The cell culture matrix can be biodegradable or non-biodegradable. Alternatively, the cell culture matrix could be made of a material that will not degrade unless it is placed into contact with a specific agent, such as an enzyme. An example of such a material would be a collagen or hyaluronan matrix that does not degrade unless it is placed into contact with collagenase or a hyaluronidase respectively. The cell chamber or pouch of the bioreactor inner microenvironment can contain one or a combination of culture medium, microbeads (e.g., polystyrene-based, poly(lactic-co-glycolic acid)-based, or poly(lactic acid)-based microbeads), hydrogel (e.g., hyaluronan-based, poly(ethylene glycol)-based, or collagen-based hydrogels), polymer matrix, tissue engineering scaffold, nanofiber (e.g., poly(lactic-co-glycolic acid)-based nanofiber), and biological extracellular matrix. The support structure can be used to promote cell adhesion and growth of the primary paracrine factor producing cells or of the supporting cells, or both. The support structure components can also be coated (e.g., with fibronectin, polylysine, integrins, cadherins, syndecans, proteins with Arg-Gly-Asp sequences, or other agents) or surface modified (e.g., with corona treatment or plasma treatment) to promote cell adhesion or cell growth. The support structure can also be used to impose a diffusion barrier to control paracrine factor release kinetics (e.g., by filling the pouch with hydrogel of varying densities). The support structure materials can be synthetic or non-synthetic, and biodegradable or non-biodegradable. Cells can be infused into the cell pouch before, during, or after infusion of the cell support environment. Alternatively, cells can be grown first in the support environment (e.g., grown on the surfaces of microspheres or nanofiber) outside the pouch and then placed into the pouch. In the present invention, the cell culture matrix comprises a hydrogel.

In accordance with some embodiments, the cell culture matrix or non-cellular support structures can be pre-formed prior to, or after, inclusion in the bioreactor enclosure with cells which have been pre-loaded and stored for later use; or alternatively, stored in its individual component reactants and then prepared only when needed, by addition directly in the bioreactor enclosure; or alternatively, formed outside the bioreactor enclosure and then injected in a formed state into the bioreactor enclosure.

In some embodiments, non-cellular support structure can also be formed, modified, or both while the device is implanted. For example, a non-cellular extracellular matrix could be formed, modified, or both in situ within the bioreactor enclosure by fibroblasts while the bioreactor is implanted. The non-cellular support structure could be modified by agents or changes in condition within the bioreactor or outside the bioreactor. For example, a hyaluronan-based matrix placed within the bioreactor enclosure can be made to degrade over time due to exposure to host hyaluronidases. Cells can be grown in the cell culture matrix inside the bioreactor enclosure, or first grown in the cell culture matrix outside the bioreactor enclosure and then injected into the bioreactor enclosure with the cell culture matrix.

As a specific example, mesenchymal stem cells are seeded on fibronectin-coated polystyrene microbeads (e.g., approximately 100 um in diameter) before placing into the pouch. The seeded cells are allowed to grow on the microbeads for a period of time before placing into the cell pouch, or seeded on the microbeads immediately before placing into the cell pouch, or, alternatively, blank microbeads are placed into the cell pouch with the cells then infused directly into the cell pouch, or some combination of the aforementioned methods. Each 100-µm diameter microbead could have a capacity of approximately 10 to 100 cells, including, for example 15, 20, 25, 30, 40, 50, 60, 70, 80, 90 to 100 cells, and the end-user can therefore control the number of cells in the cell pouch by controlling the number of microbeads infused.

It is contemplated that any cell type or combination of cell types can be seeded in the bioreactor enclosure, including but not limited to human or non-human cells, stem cells, non-stem cells, genetically modified cells, tagged cells, surface-modified cells, engineered cells, cell cluster, and prokaryotic or eukaryotic cells.

Supporting cells: The paracrine factor producing cells and the non-cellular support structures can be supported by one or more supporting cell types placed within the cell pouch, possibly allowing a longer duration of benefit provided by the paracrine factor producing cells. While these supporting cells may also produce paracrine factors, their primary purpose is to support the primary paracrine factor producing cells and generate or modulate the non-cellular support structures, possibly in response to signals from the primary paracrine factor producing cells or from the outside host. No restriction is placed on the ratio of supporting cells to paracrine factor producing cells. Examples of ratios of supporting cells to paracrine factor producing cells range from 1:1 to 1:20. Examples of supporting cell types include (but are not limited to) fibroblasts, endothelial cells, endothelial progenitor cells, stem cells, induced pluripotent stem cells, and bone marrow stromal cells. Any other suitable cell type can be used as supporting cells. Cell types can be human or non-human, stem cells, non-stem cells, genetically modified cells, tagged cells, surface-modified cells, engineered cells, and prokaryotic or eukaryotic cells.

As a first exemplary embodiment, fibroblast cells are used to enhance and regulate the formation of extracellular matrix in the pouch over time, which can enhance attachment, viability, and proliferation of paracrine factor producing cells in the pouch.

As a second exemplary embodiment, endothelial cells and endothelial progenitor cells are used to create a primitive vascular capillary system within the pouch. Such a system can increase the efficiency of mass transfer of nutrients, wastes, signaling molecules and paracrine factors across the semipermeable enclosure, and allow a denser three-dimensional packing of paracrine factor producing cells within the pouch.

As a third exemplary embodiment, bone marrow stromal cells are used to produce signaling molecules and growth factors that regulate and stimulate stem cell proliferation within the pouch. Since the subject host and the supporting cells can themselves sense signals from the growing paracrine factor producing cells within the pouch, the supporting cells can alter their activity based on the needs of the proliferating cells and the subject host and therefore continually modulate the inner microenvironment within the cell pouch.

Possible variations: The bioreactor is designed so that the end-user can customize the cell pouch inner environment at any time from the time of manufacture to the time of use.

As a first exemplary embodiment, the end-user can change the paracrine factor producing cells to change the types of paracrine factors released for different diseases (e.g., the end-user might use hepatocytes to produce factors for liver disease or mesenchymal stem cells to produce factors for heart disease).

As a second exemplary embodiment, the end-user can change the non-cellular support structure to vary the paracrine factor output or the rate of paracrine factor release (e.g., the end-user might infuse fewer cell-coated microbeads for pediatric patients than for adult patients to lower the paracrine factor output; or the end-user might decrease or increase hydrogel crosslinking density to increase or decrease, respectively, the rate of paracrine factor release to accommodate different patients or the same patient with different needs at different stages of recovery).

As a third exemplary embodiment, the end-user can change the types of supporting cells to accommodate different types of paracrine factor producing cells, or to accommodate different durations of implantation.

The end-user can also make any of these changes during the period of time that the device is implanted in the patient. For example, using an embodiment which comprises a distal port into the bioreactor, the end-user might change the paracrine factor producing cell type, supporting cells or structure, or the ratio of supporting cells to paracrine factor producing cells based on changes in the patient's condition. The end-user might wish to do this for different disease types to control the types of paracrine factors released, the total output of paracrine factors, or the release kinetics of paracrine factors.

By "hydrogel" is meant a water-swellable polymeric matrix that can absorb water to form elastic gels. On placement in an aqueous environment, dry hydrogels swell by the acquisition of liquid therein to the extent allowed by the degree of cross-linking.

Polymer is used to refer to molecules composed of repeating monomer units, including homopolymers, block copolymers, heteropolymers, random copolymers, graft copolymers and so on. "Polymers" also include linear polymers as well as branched polymers, with branched polymers including highly branched, dendritic, and star polymers.

A monomer is the basic repeating unit or units in a polymer. A monomer may itself be a monomer or may be dimer or oligomer of at least two different monomers, and each dimer or oligomer is repeated in a polymer.

In some embodiments, the cell culture matrix can comprise polymers, matrices, and gels, and the disclosure includes methods of making and using matrices, polymers and gels. One of said such polymers comprises an imide. Gels, networks, scaffolds, films and the like of interest made with the composition(s) of interest encourage cell, tissue and organ integration and growth. In other embodiments, the cell culture matrix can comprise any structure that will support cell culture (such as microbeads, nanofibers, etc.).

Biocompatible polymer, biocompatible cross-linked polymer matrix and biocompatibility are art-recognized. For example, biocompatible polymers include polymers that are neither themselves toxic to the host (e.g., and animal or human), nor degrade (if the polymer degrades) at a rate that produces monomeric or oligomeric subunits or other byproducts at toxic concentrations in the host. In certain embodiments of the present invention, biodegradation generally involves degradation of the polymer in an organism, e.g., into its monomeric subunits, which may be known to be non-toxic. Intermediate oligomeric products resulting from such degradation may have different toxicological properties, however, or biodegradation may involve oxidation or other biochemical reactions that generate molecules other than monomeric subunits of the polymer. Consequently, in certain embodiments, toxicology of a biodegradable polymer intended for *in vivo* use, such as implantation or injection into a patient, may be determined after one or more toxicity analyses. It is only necessary that the subject compositions be biocompatible as set forth above. Hence, a subject composition may comprise polymers comprising 99%, 98%, 97%, 96%, 95%, 90%, 85%, 80%, 75% or even less of biocompatible polymers, e.g., including polymers and other materials and excipients described herein, and still be biocompatible.

Cross-linked herein refers to a composition containing intermolecular cross-links and optionally intramolecular cross-links, arising from, generally, the formation of covalent bonds. Covalent bonding between two cross-linkable components may be direct, in which case an atom in one component is directly bound to an atom in the other component, or it may be indirect, through a linking group. A cross-linked gel or polymer matrix may, in addition to covalent, also include intermolecular and/or intramolecular noncovalent bonds such as hydrogen bonds and electrostatic (ionic) bonds.

"Gel" refers to a state of matter between liquid and solid, and is generally defined as a cross-linked polymer network swollen in a liquid medium. Typically, a gel is a two-phase colloidal dispersion containing both solid and liquid, wherein the amount of solid is greater than that in the two-phase colloidal dispersion referred to as a "sol." As such, a "gel" has some of the properties of a liquid (i.e., the shape is resilient and deformable) and some of the properties of a solid (i.e., the shape is discrete enough to maintain three dimensions on a two-dimensional surface).

Hydrogels can consist of hydrophilic polymers cross-linked to from a water-swollen, insoluble polymer network. Cross-linking can be initiated by many physical or chemical mechanisms. Photopolymerization is a method of covalently crosslink polymer chains, whereby a photoinitiator and polymer solution (termed "pre-gel" solution) are exposed to a light source specific to the photoinitiator. On activation, the photoinitiator reacts with specific functional groups in the polymer chains, crosslinking them to form the hydrogel. The reaction is rapid (3-5 minutes) and proceeds at room and body temperature. Photoinduced gelation enables spatial and temporal control of scaffold formation, permitting shape manipulation after injection and during gelation *in vivo.* Cells and bioactive factors can be easily incorporated into the hydrogel scaffold by simply mixing with the polymer solution prior to photogelation.

Alternatively, the reactants can contain complementary reactive groups, as an imide and an amide, that yield cross-linking without the need of an external initiator.

Hydrogels of interest can be semi-interpenetrating networks that promote cell growth. Viscosity can be controlled by the monomers and polymers used, by the level of water trapped in the hydrogel, and by incorporated thickeners, such as biopolymers, such as proteins, lipids, saccharides and the like. An example of such a thickener is hyaluronic acid or collagen.

In some embodiments, the cell culture matrix can comprise at least one monomeric unit of a biologically compatible polymer, such as chondroitin sulfate, hyaluronic acid, heparin sulfate, keratan sulfate and the like, functionalized by an imide. Those starting molecules are natural components of extracellular matrices. However, in general, any biologically compatible polymer can be used as the polymer, which polymer carries at least an imide. Other suitable polymers include those which are naturally occurring, such as a GAG, mucopolysaccharide, collagen or proteoglycan components, such as hyaluronic acid, heparin sulfate, glucosamines, dermatans, keratans, heparans, hyalurunan, aggrecan, and the like.

Cross-linked polymer matrices may include and form hydrogels. The water content of a hydrogel may provide information on the pore structure. Further, the water content may be a factor that influences, for example, the survival of encapsulated cells within the hydrogel. The amount of water that a hydrogel is able to absorb may be related to the cross-linking density and/or pore size. For example, the percentage of imides on a functionalized macromer, such as chondroitin sulfate, hyaluronic acid, dextran, carboxy methyl starch, keratin sulfate, or ethyl cellulose, may dictate the amount of water that is absorbable.

In accordance with one or more embodiments, biologically active agents can also be incorporated into the cell culture matrix in the bioreactor enclosure of the present invention. "Incorporated," "encapsulated," and "entrapped" are art-recognized when used in reference to a therapeutic agent, dye, or other material and a polymeric composition, such as the cell culture matrix composition. In certain embodiments, these terms include incorporating, formulating or otherwise including such agent into a composition that allows for sustained release of such agent in the desired application. The terms may contemplate any manner by which a therapeutic agent or other material is incorporated into a polymer matrix, including, for example, attached to a monomer of such polymer (by covalent or other binding interaction) and having such monomer be part of the polymerization to give a polymeric formulation, distributed throughout the polymeric matrix, appended to the surface of the polymeric matrix (by covalent or other binding interactions), encapsulated inside the polymeric matrix, etc. The term "co-incorporation" or "co-encapsulation" refers to the incorporation of a therapeutic agent or other material and at least one other therapeutic agent or other material in a subject composition.

More specifically, the physical form in which any therapeutic agent or other material is encapsulated in polymers, and/or in the enclosure, may vary with the particular embodiment. For example, in some embodiments, a therapeutic agent or other material may be first encapsulated in a microsphere and then combined with the polymer in such a way that at least a portion of the microsphere structure is maintained. Alternatively, a therapeutic agent or other material may be sufficiently immiscible in the polymer for use in the invention that it is dispersed as small droplets, rather than being dissolved in the polymer. Any form of encapsulation or incorporation is contemplated by the present invention, in so much as the sustained release of any encapsulated therapeutic agent or other material determines whether the form of encapsulation is sufficiently acceptable for any particular use.

In accordance with some embodiments, the cell culture matrix used within the enclosure can be bathed in liquid media containing any number of proteins, growth factors, lipids, nucleic acids, salts, any other molecules, cells, or extracellular vesicles.

Biological motifs, such as but not limited to, arginine-glycine-aspartic acid oligopeptides, collagen, and fibronectin can be incorporated into the cell culture matrix to enhance cell adhesion. Examples of classes of cell adhesion molecules include, but are not limited to, cadherins, integrins and syndecans and portions and fragments thereof.

A cell culture matrix comprising a cross-linked polymer matrix or gel and one or more biologically active agents may be prepared. The biologically active agent may vary widely with the intended purpose for the composition. The term active is art-recognized and refers to any moiety that is a biologically, physiologically, or pharmacologically active substance that acts locally or systemically in a subject. Examples of biologically active agents, that may be referred to as "drugs", are described in well-known literature references such as the Merck Index, the Physicians' Desk Reference, and The Pharmacological Basis of Therapeutics, and they include, without limitation, medicaments; vitamins; mineral supplements; substances used for the treatment, prevention, diagnosis, cure or mitigation of a disease or illness; substances which affect the structure or function of the body; or pro-drugs, which become biologically active or more active after they have been placed in a physiological environment. A specific example of a "drug" is heparin, which could be used for its anti-thrombotic effect. Various forms of a biologically active agent may be used which are capable of being released for example, into adjacent tissues or fluids upon administration to a subject. In some embodiments, a biologically active agent may be used in cross-linked polymer matrix for use in this invention to, for example, promote angiogenesis. In other embodiments, a biologically active agent may be used in cross-linked polymer matrix for use in this invention, to treat, ameliorate, inhibit, or prevent a disease or symptom, in conjunction with, for example, promoting angiogenesis or healing after an insult or injury.

Further examples of biologically active agents include, without limitation, enzymes, receptor antagonists or agonists, hormones, growth factors, autogenous bone marrow, antibiotics, antimicrobial agents, and antibodies. The term "biologically active agent" is also intended to encompass various cell types and genes that can be incorporated into the compositions for use in the invention.

In certain embodiments, the subject compositions comprise about 1% to about 75% or more by weight of the total composition, alternatively about 2.5%, 5%, 10%, 20%, 30%, 40%, 50%, 60% or 70%, of a biologically active agent.

Non-limiting examples of biologically active agents include the following: adrenergic blocking agents, anabolic agents, androgenic steroids, anti-cholesterolemic and anti-lipid agents, anti-cholinergics and sympathomimetics, anti-coagulants, anti-hypertensive agents, anti-infective agents, anti-inflammatory agents such as steroids, non-steroidal anti-inflammatory agents, anti-pyretic and analgesic agents, anti-thrombotic agents, anti-anginal agents, biologicals, cardioactive agents, vasodilators, coronary dilators, diagnostic agents, erythropoietic agents, estrogens, growth factors, peripheral vasodilators, progestational agents, prostaglandins, vitamins, antigenic materials, and prodrugs.

Further, recombinant or cell-derived proteins may be used, such as recombinant beta-glucan; bovine immunoglobulin concentrate; bovine superoxide dismutase; formulation comprising fluorouracil, epinephrine, and bovine collagen; recombinant hirudin (r-Hir), HIV-1 immunogen; recombinant human growth hormone, recombinant EPO (r-EPO); gene-activated EPO (GA-EPO); recombinant human hemoglobin (r-Hb); recombinant human mecasermin (r-lGF-l); recombinant interferon α; lenograstim (G-CSF); olanzapine; recombinant thyroid stimulating hormone (r-TSH); and topotecan.

Still further, the following listing of peptides, proteins, and other large molecules may also be used, such as interleukins 1 through 18, including mutants and analogues; interferons α, γ, and which may be useful for cartilage regeneration, hormone releasing hormone (LHRH) and analogues, gonadotropin releasing hormone, transforming growth factor (TGF); fibroblast growth factor (FGF); tumor necrosis factor-α); nerve growth factor (NGF); growth hormone releasing factor (GHRF), epidermal growth factor (EGF), connective tissue activated osteogenic factors, fibroblast growth factor homologous factor (FGFHF); hepatocyte growth factor (HGF); insulin growth factor (IGF); invasion inhibiting factor-2 (IIF -2); bone morphogenetic proteins 1-7 (BMP 1-7); somatostatin; thymosin- α-γ-globulin; superoxide dismutase (SOD); and complement factors, and biologically active analogs, fragments, and derivatives of such factors, for example, growth factors.

Members of the transforming growth factor (TGF) supergene family, which are multifunctional regulatory proteins, may be incorporated in a polymer matrix for use in the present invention. Members of the TGF supergene family include the beta transforming growth factors (for example, TGF-131, TGF-132, TGF-133); bone morphogenetic proteins (for example, BMP-1, BMP-2, BMP-3, BMP-4, BMP-5, BMP-6, BMP-7, BMP-8, BMP-9); heparin-binding growth factors (for example, fibroblast growth factor (FGF), epidermal growth factor (EGF), platelet-derived growth factor (PDGF), insulin-like growth factor (lGF)), (for example, inhibin A, inhibin B), growth differentiating factors (for example, GDF-l); and Activins (for example, Activin A, Activin B, Activin AB). Growth factors can be isolated from native or natural sources, such as from mammalian cells, or can be prepared synthetically, such as by recombinant DNA techniques or by various chemical processes. In addition, analogs, fragments, or derivatives of these factors can be used, provided that they exhibit at least some of the biological activity of the native molecule. For example, analogs can be prepared by expression of genes altered by site-specific mutagenesis or other genetic engineering techniques.

In accordance with an embodiment, a thiol-modified hyaluronan hydrogel incorporating collagen can be used as the cell culture matrix for stem cells within the bioreactor enclosure. In this specific embodiment, a suspension of stem cells is prepared in the thiol-modified hyaluronan solution, infused into the bioreactor enclosure, and crosslinked with thiol-reactive polyethylene glycol diacrylate to form the hydrogel.

In accordance with another embodiment, cells could be grown on the surface of microparticles outside the bioreactor enclosure; the microparticles coated with cells could then be injected into the bioreactor enclosure and fill a portion or a majority of the inner volume of the bioreactor enclosure.

Catheter Design: (a) Structure. In accordance with some embodiments, the present invention can comprise a catheter. The catheter of the present invention can comprise one tube, or alternatively, a multitude of tubes which are joined at one or more points. Each catheter tube can have one or multiple lumens that provide options for infusion, sampling, or circulation of cells, media, and other materials. In some embodiments, one or more of the catheter tubes can comprise one or more additional ports, with some of these ports opening into the inner lumen of the cell pouch, and others directly in communication with the circulation to enable its use as an intravascular line. For example, one of the lumens can be dedicated for guide wire passage in an "over-the-wire" or in a monorail "rapid exchange" configuration to facilitate intravascular placement and positioning. Another lumen can connect to an inflatable air- or fluid-filled balloon placed at the distal end of the catheter tube to aid in positioning. The catheter is attached to one or more external ports allowing infusion, sampling, and circulation of cells, media and other materials as necessary. (b) Material. The catheter housing can be manufactured from any suitable biocompatible material (for example, polyvinyl chloride, polyesters, polycarbonates, polyurethanes, polyamides, polyimides, fluoropolymers, polyolefins, or polyetheretherketone). The exterior of the catheter can be coated with molecules which help enhance cell attachment and function. Some of these molecules include poly-L-lysine, fibronectin, or other proteins with Arg-Gly-Asp sequences. This can be accomplished by first oxidizing the catheter surface in a plasma reactor or with chemical oxidizing agents such as potassium permanganate, then reacting the surface with the appropriate molecular functional group. The exterior of the catheter can also be spray- or dip-coated with an anti-coagulant (such as heparin) to minimize thrombus formation during implantation. The exterior of the catheter may also be surface-modified (e.g., using fluoropassivation) to minimize fibrosis or to facilitate delivery. Alternatively, a portion of the catheter can be modified to improve cell adhesion (e.g., the segment of the catheter spanning the inside of the cell pouch), while another portion of the catheter is modified to reduce fibrosis, thrombosis and facilitate delivery (e.g., the segment of the catheter outside of the cell pouch). Additionally, drug delivery devices can be attached to the catheter. Such devices can include, for example, hydrogels, polymeric matrices, beads, and nanoparticles comprising drugs or biologically active agents.

In accordance with some embodiments comprising a catheter, the catheter housing can made of polyvinyl chloride tubing (or any other suitable biocompatible material) composed of a multitude of lumens, proximal access ports and distal apertures, similar to standard multi-lumen central venous catheters. These ports can be used for circulation of cells, media, therapeutic agents, gas, or any other agent recognized by one of ordinary skill in the art to be beneficial. Several optional modifications to the basic tube configuration would be apparent to one of ordinary skill in the art.

In some embodiments, the surface of the catheter upon which the bioreactor is mounted can be coated with molecules which help enhance stem cell attachment and function. Some of these molecules include polylysine, fibronectin, or other proteins with Arg-Gly-Asp sequences. This can be accomplished by first oxidizing the catheter surface in a plasma reactor or with chemical oxidizing agents such as potassium permanganate, then reacting the surface with the appropriate molecular functional group. The exterior of the catheter can also be spray- or dip-coated with an anti-coagulant (such as heparin) to minimize thrombus formation during and following implantation.

Guiding balloon: A small balloon (~ 1 cm in diameter) can be placed near the tip of the catheter, which upon filling with gas or a fluid, can assist in guiding intravascular placement.

Guidewire guidance: A separate lumen within the catheter can be used to pass over a guide wire in an "over-the-wire" or a monorail "rapid exchange" configuration to guide intravascular placement The device would also allow for infusion, intermittent recycling or continuous circulation of cells, cell-conditioned media, concentrated paracrine factors, or any other therapeutic fluids that may have or are determined to have a beneficial effect

In some embodiments the catheter-based device can be removed when desired. The enclosure is first deflated by drawing back through the infusion port. The entire device can then be pulled out of the body. The vascular sheath is removed, and manual compression or vascular closure devices are used to achieve hemostasis.

Secondary device-mounted bioreactor. In an embodiment, bioreactor enclosures as described above can be attached to various other secondary devices and implanted along with the device. The bioreactor enclosures can be miniaturized as needed to attach to the secondary device. In the cardiovascular arena, secondary devices include, but are not limited to, stents, balloons, intra-aortic balloon pumps, percutaneous and surgically implanted ventricular assist devices, percutaneous and surgically implanted prosthetic valves and valve clips or rings, endovascular grafts, thrombus filters, pacemaker or defibrillator surfaces or leads, septal occluders, atrial appendage closure devices, pulmonary artery catheters, venous catheters, and arterial catheters, among others. Outside the cardiovascular arena, any device conferring either direct or indirect access to a target tissue is a possible candidate for attaching a bioreactor enclosure.

In accordance with some embodiments, cells within the bioreactor can be loaded within a three-dimensional network of nanofibers, termed a "nanofiber cloud." A nanofiber is defined as a fiber with a typical diameter of approximately 1000 nm, and can be made by known methods such as electrospinning. The nanofibers can be made from most biologically compatible polymers, including, for example, poly-L-lactic acid copolymers. The polymer is then electrospun onto a matrix or surface and the fibers collected. The inventors have published data showing that stem cells easily adhere to and proliferate vigorously on matrices composed of nanofibers (Biomaterials 2015 Jun; 52:318-26).

As such, an example provides an intravascular implantable bioreactor, comprising cells enclosed within a three-dimensional network of nanofibers within an enclosure, said cells being capable of producing paracrine factors, wherein the enclosure can be collapsible and expandable to be intravascularly implantable, wherein the enclosure is semi-permeable such that it provides containment of the cells preventing the egress of the cells while further providing an immunological barrier.

In some embodiments, the nanofiber cloud can be formed inside the bioreactor enclosure, or alternatively, formed outside the bioreactor enclosure and then later injected or placed into the bioreactor enclosure. The nanofiber cloud can fill the bioreactor enclosure either entirely or partially.

It will be understood by those of ordinary skill in the art that the nanofiber density or nanofiber orientations of the nanofiber cloud in the bioreactor pouch can be modulated depending on desired cloud permeability, cell seeding density, and cell seeding pattern.

The nanofibers can be based on biodegradable polymer materials including but not limited to poly(lactic-co-glycolic acid), polycaprolactone, polylactic acid, or polydioxanone. Nanofibers can also be composed of non-biodegradable materials including but not limited to polyurethane, polytetrafluoroethylene, polyethylene terephthalate, or polysulfone. Nanofibers can also be composed of biological materials including but not limited to cellulose, collagen, lipids, nucleic acids, and proteins. Nanofibers can also be composed of any combination of one or more biodegradable, non-biodegradable, and biological materials.

"Biodegradable" is art-recognized, and includes monomers, polymers, polymer matrices, gels, compositions and formulations, such as those described herein, that are intended to degrade during use, such as *in vivo.* Biodegradable polymers and matrices typically differ from non-biodegradable polymers in that the former may be degraded during use. In certain embodiments such use involves *in vivo* use, such as *in vivo* therapy, and in other certain embodiments, such use involves *in vitro* use. In general, degradation attributable to biodegradability involves the degradation of a biodegradable polymer into its component subunits, or digestion, e.g., by a biochemical process, of the polymer into smaller, non-polymeric subunits. In certain embodiments, two different types of biodegradation may generally be identified. For example, one type of biodegradation may involve cleavage of bonds (whether covalent or otherwise) in the polymer backbone. In such biodegradation, monomers and oligomers typically result, and even more typically, such biodegradation occurs by cleavage of a bond connecting one or more of subunits of a polymer. In contrast, another type of biodegradation may involve cleavage of a bond (whether covalent or otherwise) internal to a side chain or that connects a side chain, functional group and so on to the polymer backbone. For example, a therapeutic agent, biologically active agent, or other chemical moiety attached as a side chain to the polymer backbone may be released by biodegradation. In certain embodiments, one or the other or both general types of biodegradation may occur during use of a polymer. As used herein, the term "biodegradation" encompasses both general types of biodegradation.

The degradation rate of a biodegradable polymer often depends in part on a variety of factors, including the chemical identity of the linkage responsible for any degradation, the molecular weight, crystallinity, biostability, and degree of cross-linking of such polymer, the physical characteristics of the implant, shape and size, and the mode and location of administration. For example, the greater the molecular weight, the higher the degree of crystallinity, and/or the greater the biostability. The term "biodegradable" is intended to cover materials and processes also termed "bioerodible."

In certain embodiments, the biodegradation rate of such polymer may be may depend on the presence of enzymes, for example, a chondroitinase. In such circumstances, the biodegradation rate may depend on not only the chemical identity and physical characteristics of the polymer matrix, but also on the identity of any such enzyme.

In certain embodiments, polymeric formulations biodegrade within a period that is acceptable in the desired application. In certain embodiments, such as *in vivo* therapy, such degradation occurs in a period usually less than about five years, one year, six months, three months, one month, fifteen days, five days, three days, or even one day on exposure to a physiological solution with a pH between 6 and 8 having a temperature of between about 25 °C and 37 °C. In other embodiments, the polymer degrades in a period of between about one hour and several weeks, depending on the desired application. In some embodiments, the polymer or polymer matrix may include a detectable agent that is released on degradation.

In some embodiments, adhesion molecules such as arginine-glycine-aspartic acid oligopeptides, collagen, and fibronectin, for example, can be incorporated into the nanofiber cloud to enhance cell adhesion.

Cells for use in the bioreactor can be seeded in the nanofiber cloud immediately after electrospinning and stored for later use, or alternatively, seeded in the nanofiber cloud immediately prior to use.

In accordance with some embodiments, any cell type or combination of cell types can be seeded in a nanofiber cloud within the bioreactor enclosure, including but not limited to human or non-human cells, stem cells, non-stem cells, genetically modified cells, tagged cells, surface-modified cells, engineered cells, prokaryotic, or eukaryotic cells.

It will be understood by those of skill in the art that the nanofiber cloud can be bathed in liquid media containing any number of proteins, growth factors, lipids, nucleic acids, salts, any other molecules, cells, or extracellular vesicles. The nanofiber cloud can also be bathed in a culture matrix, including but not limited to that described herein such as a hydrogel. The nanofiber cloud can also be bathed in any combination of liquid media and culture matrix such as a hydrogel.

It will also be understood by those of ordinary skill that one or more types of cells not directly contributing to bioreactor paracrine factor production can be incorporated into the bioreactor pouch. As an example, such cells can be used within the bioreactor enclosure to enhance the formation of extracellular matrix within the enclosure, or to support the viability of other cells in the bioreactor enclosure. It is contemplated that any cell type can be used. As examples, cell types can be human or non-human, stem cells, non-stem cells, genetically modified cells, tagged cells, surface-modified cells, engineered cells, cell clusters, prokaryotic or eukaryotic cells.

In accordance with an embodiment, an example provides a bioreactor enclosure which contains, for example, fibroblasts to support extracellular matrix formation inside the bioreactor pouch that will enhance attachment, viability, and proliferation of the primary paracrine factor-producing bioreactor cells.

Alternative compositions of the bioreactor enclosure.

As previously detailed above, the bioreactor enclosure can be constructed from a wide spectrum of semi-permeable membranes, biologic or synthetic, with pre-defined molecular weight cut-offs designed to restrict movement of cells, but allow free transfer of paracrine factors, nutrients, and wastes.

### Machined materials.

The bioreactor enclosure can be manufactured from any non-permeable material by machining the material to form pores of appropriate diameters and density. The bioreactor enclosure can also be manufactured from any intrinsically semi-permeable material (but with an inappropriate pore diameter or density) by machining the material to form pores of the appropriate diameters and density.

As used herein, an "appropriate pore diameter" is defined as one that will prevent cell passage while allowing free passage to paracrine factors including exosomes, as well as nutrients, wastes, and fluids.

As used herein, an "appropriate pore density" is defined as one that is sufficient to support viability of the cells within the bioreactor enclosure, and can vary depending on the maximal cell population and metabolic demands of the cells within the bioreactor pouch. As an example, pore diameters ranging from 100 nm to 5000 nm could satisfy these criteria. As an example, pore densities ranging from 100,000 to 100 million pores per square centimeter could satisfy these criteria.

In accordance with some embodiments, a continuous or intermittent outward fluid flux across the bioreactor enclosure can be applied to favor egress of materials from the pouch and reduce ingress of materials into the enclosure. A continuous or intermittent inward fluid flux across the bioreactor enclosure can be applied for the opposite effect. Such a fluid flux can be applied to any of the enclosures described in this disclosure. As a specific example, a continuous outward flux of normal saline at a rate of 10 cc/hr applied by connecting the lumen of the bioreactor enclosure to an external fluid source. Additionally and optionally, the fluid can include any agent needed by the patient (such as medications, anti-thrombotic agents, anti-inflammatory agents, nutrition), or any agent needed by the cells or supporting structures within the bioreactor pouch. A fluid flux can be created using a peristaltic pump, IV infusion pump and other pumps used in medical devices known in the art.

Methods used for machining the pouch include but are not limited to track-etching, microdrilling, laser-drilling, photolithographic etching, or any other suitable means to form pores. Track-etching is a technique in which ions are used to bombard the material and form damage tracks; pores are then created by enlarging the damage tracks using a chemical etchant, such as sodium hydroxide. Microdrilling and laser-drilling are techniques wherein pores are physically formed using micro-drills or lasers. Photolithographic etching is a well-established technique from the semiconductor industry wherein the geometric pattern of pores is transferred through a photomask to an enclosure surface coated with photoresist; a liquid wet-etch or plasma dry-etch process is then used to produce the pores.

In some embodiments, enclosure materials can be composed of non-biodegradable materials including but not limited to polyurethane, polytetrafluoroethylene, polysulfone, or polyethylene terephthalate. Enclosure materials can also be composed of biodegradable materials including but not limited to poly(lactic-co-glycolic acid), polycaprolactone, polylactic acid, or polydioxanone. Enclosure materials can also be composed of biological materials including but not limited to cellulose, collagen, lipids, nucleic acids, and proteins. Enclosure materials can also be composed of any combination of one or more biodegradable, non-biodegradable, and/or biological materials. Enclosure materials can be coated with molecules which enhance cell attachment and function. Specific examples include fibronectin, polylysine, integrins, cadherins, syndecans, and proteins with Arg-Gly-Asp sequences. Enclosure materials can also be surface-modified to enhance cell attachment and function. Specific examples of this process include corona-treatment and plasma-treatment. Enclosure materials can also be coated or surface-modified to minimize thrombosis or fibrosis, reduce immunogenicity, improve biocompatibility, or other substances to improve deliverability. Specific examples include attaching heparin chains or polyethylene glycol chains or fluoropassivation treatment. The adjustment of coatings or surface modification is well known to those of ordinary skill in the art. Enclosure materials can include drug delivery capacity (e.g., through the use of hydrogel or polymer matrices incorporating drugs, biologics, or other agents), or be attached to drug delivery devices.

It will be understood by those of skill in the art, that as described above for the non-machined bioreactor enclosure, the machined bioreactor enclosure may be formed with surface undulations, crevices, or both, or neither. In some embodiments, the machined enclosure can be expandable or collapsible, or both, or neither. In some embodiments, the enclosure may be coated with agents on its external surface that impede cell adhesion or thrombus formation, with agents on its internal surface that enhance cell adhesion, or both, or neither.

In accordance with an embodiment, an expandable and collapsible semi-permeable bioreactor enclosure can be formed from impermeable polyethylene terephthalate membranes by track-etching pores of approximately 1000 nm to 2500 nm diameter at a pore density of approximately 3 million to 10 million pores per square centimeter.

Composites of permeable membranes and semi-permeable materials.

In accordance with an embodiment, the present invention provides an implantable bioreactor, comprising cells within an enclosure, said cells being capable of producing paracrine factors, wherein the enclosure can be collapsible and expandable, wherein the enclosure comprises at least one semi-permeable layer of material in contact or adjacent to at least one permeable layer of material and wherein the permeable layer of material is on the outward facing side of the enclosure, and the enclosure is semi-permeable such that it provides containment of the cells preventing the egress of the cells while further providing an immunological barrier.

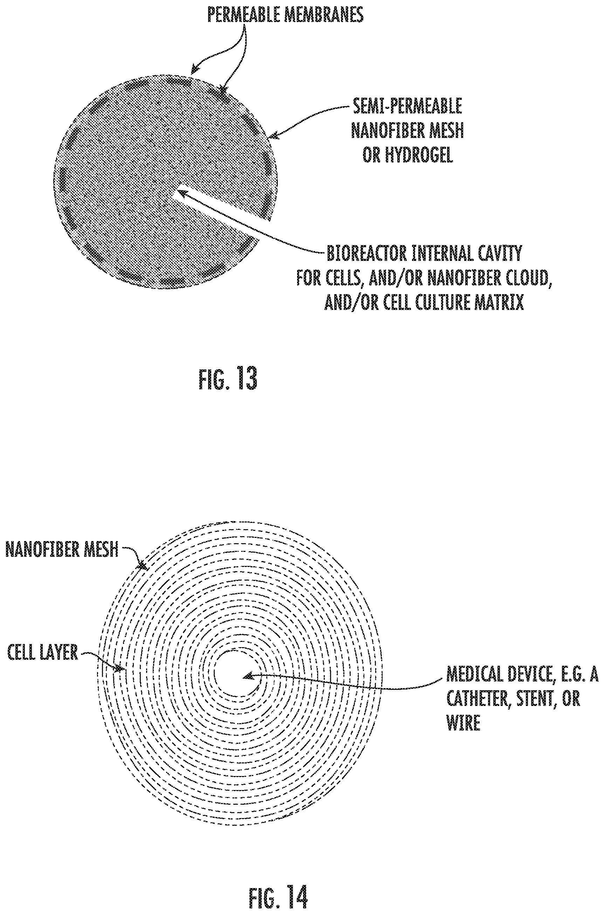

In accordance with another embodiment, the present invention provides an implantable bioreactor, comprising cells within an enclosure, said cells being capable of producing paracrine factors, wherein the enclosure can be collapsible and expandable, wherein the enclosure comprises at least one semi-permeable layer of material located in between and in contact or adjacent to at least a first and second permeable layer of material, wherein the first permeable layer of material is on the outward facing side of the enclosure, and the second layer of permeable material is on the inward facing side of the enclosure, and wherein the layer is semi-permeable such that it provides containment of the cells preventing the egress of the cells while further providing an immunological barrier (Fig. 13).

Another example provides appropriately semi-permeable bioreactor enclosures which can be manufactured by sandwiching appropriately semi-permeable or machined materials between layers of more permeable membranes, or, alternatively, attaching one semi-permeable or machined material to a permeable membrane to comprise a composite enclosure.

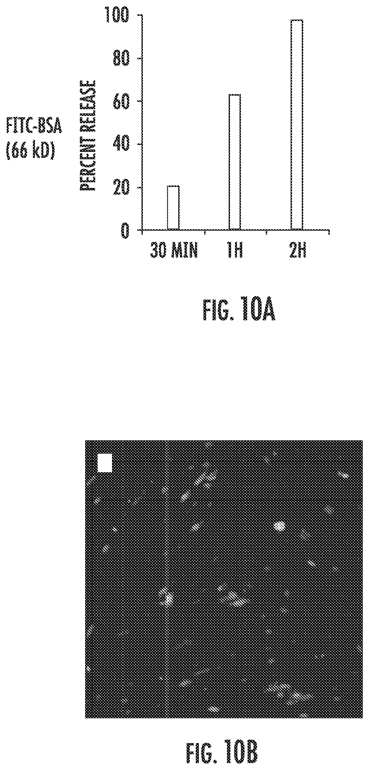

In accordance with another embodiment, two layers of permeable membranes each with average pore diameters in excess of 5000 nm in an outer layer can be used to sandwich a nanofiber mesh with an average pore diameter of less than 5000 nm in an inner layer (Figure 10). The nanofiber mesh can be composed of one or more biodegradable, non-biodegradable, biological, or non-biological materials to comprise a composite enclosure.

In accordance with another embodiment, two layers of permeable membranes in the outer layers each with average pore diameters in excess of 5000 nm can be used to sandwich a hydrogel with inner layers having an average pore diameter of less than 5000 nm (Figure 10) to comprise a composite enclosure. The hydrogel can be composed of biodegradable, non-biodegradable, biological, or non-biological materials.

In accordance with some other embodiments, one, two, or more layers of such permeable membranes can be used in the composite enclosure. The composite enclosure may be formed with surface undulations, crevices, or both, or neither.

In some embodiments, the composite enclosure may be expandable or collapsible, or both, or neither.

In some embodiments, the composite enclosure may be coated with agents on its external surface that impede cell adhesion or thrombus formation, with agents on its internal surface that enhance cell adhesion, or both, or neither.

### Folded enclosure bioreactor.

In accordance with an embodiment, the present invention provides an implantable bioreactor, comprising cells enclosed within an enclosure, said cells being capable of producing paracrine factors, wherein the enclosure comprises at least one semi-permeable layer of material in contact or adjacent to at least one layer of cells within a cell culture matrix and wherein the first permeable layer of material is on the outward facing side of the enclosure, and the layer of cells is on the inward facing side of the enclosure, and is capable of being rolled or folded over on itself such that the semi-permeable layer provides containment of the cells preventing the egress of the cells while further providing an immunological barrier. Such an embodiment could allow the bioreactor to be placed into any body cavity, organ, blood vessel, tissue, or on the skin, or within an external or internal wound, or within a surgical wound.

In some embodiments, the folded enclosure bioreactor can be comprised of layers of machined materials having a defined porosity.

In some embodiments, the bioreactor comprises one or multiple different semi-permeable and/or machined material layers and/or permeable layers, and can be manufactured from one or a multitude of different permeable and semi-permeable sheets by placing the sheets one atop the other, and rolling them up or arranging them around a central axis so as to form a spiral pattern in cross-section, much as in the structure of a "Swiss roll" as shown in Fig. 14. In a variation, instead of rolling up the sheets, the sheets can be folded into one of any desirable configuration, such that the cells are sandwiched between semi-permeable sheets.

In some embodiments, the different permeable and/or semi-permeable sheets of the bioreactor can be rolled up or folded up to incorporate or circumscribe a cavity or tube structure around a central axis, to accommodate cells with or without culture media, culture matrix or nanofiber cloud. Alternatively, the different permeable and/or semi-permeable sheets of the bioreactor can be rolled up or folded up directly onto or around the surface of a medical device such as a stent or a catheter.

For example, in an embodiment, the different permeable and/or semi-permeable sheets of the bioreactor are rolled up with a catheter at the center of the central cavity. In some embodiments, the catheter can be made of porous materials.

In accordance with some embodiments, one or more types of cells can be grown directly on one or more of the different permeable and/or semi-permeable sheets of the bioreactor, before being rolled up or folded up together with the permeable and/or semi-permeable sheets.

In an embodiment, the cell layers can be grown on flat sheets of biodegradable nanofiber, rolled up, and become incorporated into the spiral structure that can comprise part of a bioreactor.

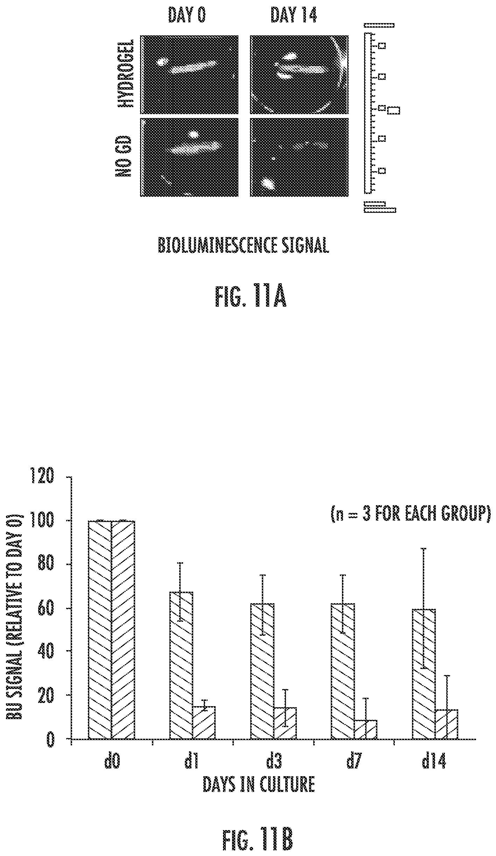

In another embodiment, one flat sheet of semi-permeable nanofiber mesh and can be cultured with stem cells to confluence and rolled up against a catheter, forming a spiral of concentrically alternating stem cells and semi-permeable nanofiber mesh layers surrounding the catheter at its center (Fig. 11).

In some embodiments, the rolled bioreactor can be formed with surface undulations, crevices, or both.

In some embodiments, the rolled bioreactor can be expandable or collapsible, or both.

In some embodiments, the rolled bioreactor can be coated with agents that affect cell adhesion or thrombus formation.

In accordance with an alternative embodiment, the rolled bioreactor can comprise specific cell types that are grown in specific portions of the permeable and/or semi-permeable sheets in such a manner that when the permeable and/or semi-permeable sheets are rolled up or folded up, a desired three-dimensional structure containing cells in a specific configuration is formed. This configuration can provide a convenient method of creating a three-dimensional tissue engineering scaffold from the permeable and/or semi-permeable sheets.

For example, an embodiment can provide a useful structure that can be used to create an artificial blood vessel. A long flat strip of a semi-permeable material having at least enough length such that it comprises three or more segments, wherein each segment has a length sufficient to circumscribe the axis of the spiral once, is seeded with endothelial cells in its first segment, smooth muscle cells in its middle segment, and fibroblasts in its last segment. The composite structure is then rolled up, thereby creating a structure similar in construct to a blood vessel, with endothelial cells in the innermost layer, smooth muscle cells in the middle layers, and fibroblasts in the outermost layers.

It will be understood by those of skill in the art, that in all of these embodiments, cells can be seeded on the permeable and/or semi-permeable sheets either before or after the permeable and/or semi-permeable sheets have been folded up or rolled up. Alternatively, cells can be seeded during the folding or rolling process.

### EXAMPLES

### EXAMPLE 1

In Vitro and In Vivo Testing of First Generation (G1) Stem Cell Implantable Bioreactor (SCIB).

We fabricated and tested prototypes of a first generation SCIB (G1-SCIB) with a cylindrical stem cell chamber (Fig. 1) attached to a vascular catheter shaft. The G1 prototype was constructed from a semi-permeable cellulose ester membrane with a 100-kilodalton molecular weight cut-off, and as such, is impermeable to the exosomal fraction of paracrine factors (PFs). A 10-cm membrane segment was attached to a 20-cm modified 6 French vascular catheter (Boston Scientific, Marlborough, MA) with a heat-sealed distal end and the shaft fenestrated at 1-cm intervals using an 18-Gauge needle. The membrane was secured to the mid-portion of the modified catheter using cyanoacrylate medical device adhesive (Henkel, Rocky Hill, CT), 2-0 silk surgical suture, and 0.125" medical grade heat shrink tubing (InsulTab, Woburn, MA). The SCIBs were sterilized in 70% ethanol under ultra violet light for 12 hours and washed in phosphate buffered saline (PBS; Mediatech, Manassas, VA), and cell culture media before cell culture experiments and *in vivo* implantation. As designed, the device is deployed with the stem cell chamber in the lumen of a large blood vessel to allow for free exchange of PFs, signaling molecules, nutrients, and wastes with the blood stream.

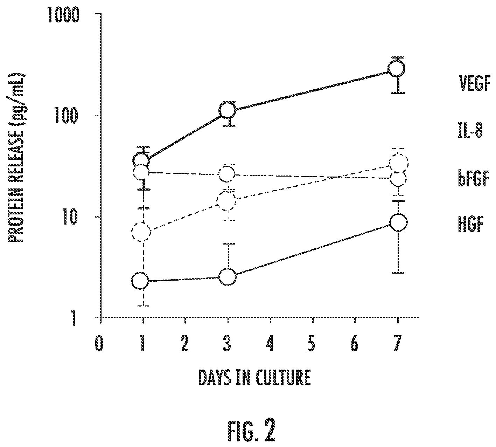

G1-SCIB MSC Viability and Paracrine Factor (PF) Release. To assess *in vitro* PF release, SCIBs were loaded with increasing numbers of human mesenchymal stem cells (MSCs, 10⁵, 10⁶, and 5 x 10⁶) and submerged in culture media in separate flasks for 7 days at typical culture conditions (37 °C, 5% CO₂). The culture medium consisted of Alpha-MEM (Mediatech, Manassas, VA) supplemented with 20% fetal bovine serum (FBS; Hyclone, Logan, UT), L-glutamine (350 µg/mL; Mediatech, Manassas, VA), and penicillin/streptomycin (50 iU/mL / 50 µg/mL, Mediatech, Manassas, VA). Samples of media outside the SCIBs were collected on days 1, 3, and 7, centrifuged to pellet any debris, then flash frozen at -80C. The concentrations of vascular endothelial growth factor (VEGF), basic fibroblast growth factor (bFGF), hepatocyte growth factor (HGF), and interleukin-8 (IL-8) in this conditioned media (CM) were assessed using multiplex ELISA (Quansys, Logan, UT). SCIBs loaded with media alone (i.e. no cells) were used as controls. MSCs showed high viability over 7 days in culture (75.4 ± 11.6%; n=6), and produced relevant PFs as assessed by ELISA (Fig. 2). MSCs in SCIBs placed in a hypoxic chamber increased angiogenic PF release, including VEGF, confirming the ability of contained cells to alter their PF release based on conditions external to the SCIB. To determine the MSC capacity of the G1-SCIB, escalating doses of MSCs were cultured in the stem cell chamber pouch over 7 days, and PF production was assessed; PF production of the G1-SCIB peaked with a cell dose of 25 million cells, and this dose was taken as the maximal capacity of the G1-SCIB.

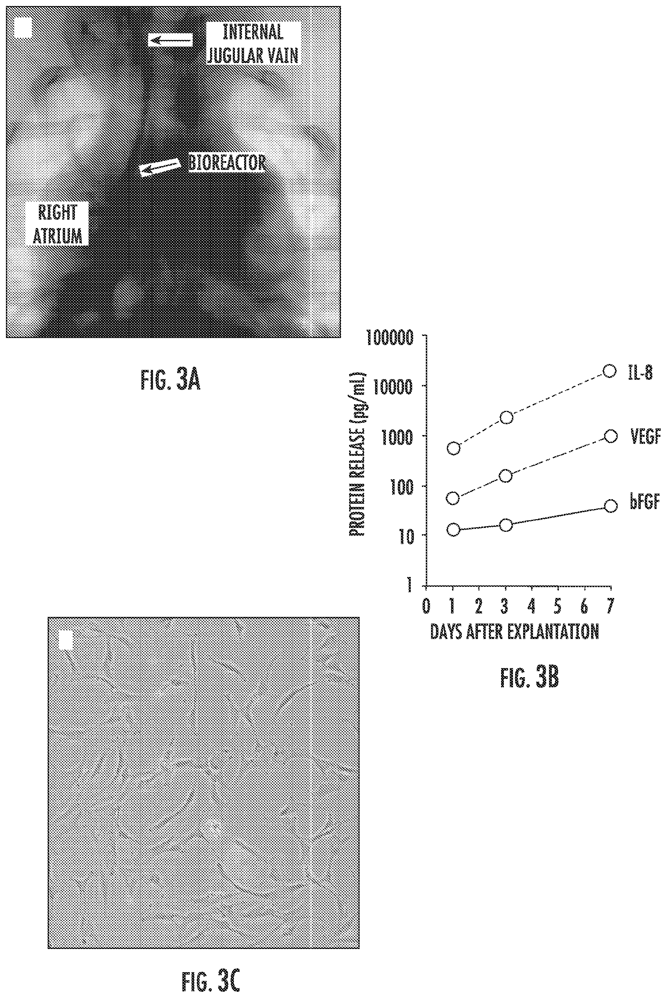

G1-SCIB Biocompatibility and Immunoprotection. To confirm biocompatibility of the device in vivo, SCIBs were inserted in the right internal jugular vein in Yorkshire swine (20-30 kg) via surgical cut-down and advanced to the junction of the SVC and right atrium under fluoroscopic guidance. Once in place, the proximal end of the SCIB was secured and the wound closed. G1-SCIBs loaded with 10⁶ human MSCs were implanted in the superior vena cava of pigs (Fig. 3A), and despite the use of xenogeneic MSCs, there was no evidence of immune or transfusion reaction. After 1 week, the G1-SCIBs were explanted and placed back in culture. MSCs recovered from the explanted G1-SCIBs showed continued growth, continued release of representative PFs such as VEGF over a subsequent 7 days (Fig. 3B), and normal morphology (Fig. 3C).

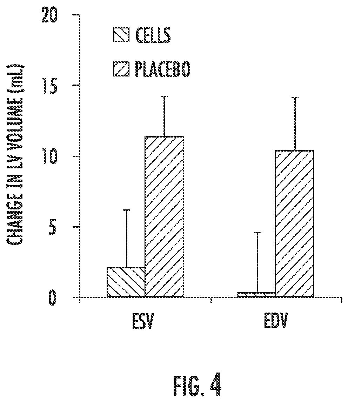

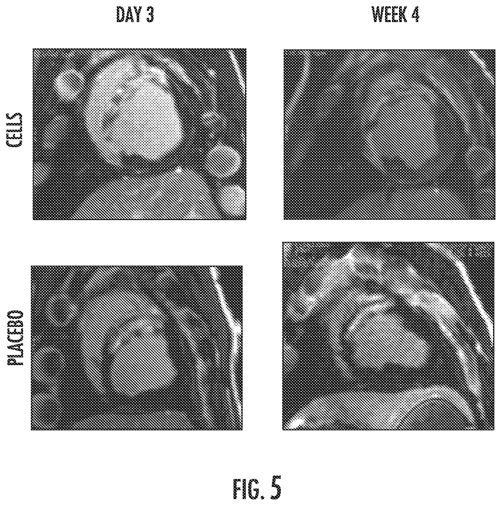

Placebo-Controlled Pig Myocardial Infarction Study. To determine whether SCIB-based therapy altered adverse cardiac remodeling after myocardial infarction (M), G1-SCIBs containing 25 million pig MSCs vs. G1-SCIBs containing media alone (placebo) were deployed in the superior vena cava (SVC) in pigs 3 days after anterior MI induced by 90 minutes of left anterior descending artery occlusion (n=8 in each group). Baseline cardiac MRI was obtained just prior to SCIB insertion 3 days after MI using a whole body 3.0T MR scanner to assess left ventricular (LV) function, chamber size, and scar prior to implantation of a bioreactor. Cine images were acquired using a radiofrequency spoiled gradient recalled echo (SPGR) pulse sequence (repetition time, TR=4.3msec, echo time, TE=2.1msec; flip α=12°; in-plane resolution 1.3 x 1.3mm; 30 phases/cardiac cycle). Images for late gadolinium enhancement (LGE) were acquired after peripheral injection of Magnevist 0.2 mmol/kg (Bayer, Wayne, NJ) with an inversion recovery-prepared T1 weighted gradient echo sequence (TR=5.3; TE=2.6; 1 R-R; flip α=20°; in-plane resolution 1.4 x 1.5mm). Short axis images from base to apex were acquired with 6mm slice thickness and no gap for cine and LGE images. Cardiac MRI at 4 weeks using identical imaging parameters showed markedly reduced adverse left ventricular cardiac remodeling in cell-treated pigs compared to placebo pigs (by blinded analysis) while the G1-SCIB was in place, with smaller increases in end-systolic volume (ESV, 2.1 ± 4.1mL vs. 11.4 ± 2.9mL, cells vs. placebo, p=0.036) and end-diastolic volume (EDV, 0.3 ± 4.3mL vs. 10.4 ± 3.8mL, cells vs. placebo, p=0.059) (Fig 4). Left ventricular ejection fraction decreased in placebo pigs (-5.8 ± 1.7%, p=0.03) but not in cell-treated pigs (-2.3 ± 2.9%, p=0.33). Both groups had change in total scar size as assessed by late gadolinium-enhanced cardiac MRI (Fig. 5). Scar size was further compartmentalized into core dense scar and heterogeneous gray scar. Core extent comprised all pixels with SI>50% of maximal SI within the hyper-enhanced region. Regions of microvascular obstruction were included as part of the core. Gray zone extent comprised all pixels with SI greater than peak SI in normal myocardium, but <50% of maximal SI within the hyper-enhanced region. When the components of the heterogeneous scar mass (i.e. the "gray" and dense "core" masses) were analyzed as such, animals receiving SCIB-based cell therapy showed a significant increase in heterogeneous gray scar coupled with a significant decrease in dense core scar that was not present in animals receiving placebo. G1-SCIBs removed from animals were placed in culture and those with MSCs continued to release PFs (787 ± 257 pg/mL VEGF at 7 days). Importantly, all animals survived the duration of the study; there was no late or unexpected mortality to suggest lethal arrhythmia, device thrombosis, or pulmonary embolus.

Safety. All pigs implanted with SCIBs survived the entire study. Despite the use of allogeneic pig MSCs, there was no evidence of fever or leukocytosis that would suggest an immune reaction. Pigs were observed for 4 weeks after SCIB removal and remained asymptomatic. On necropsy, no evidence of neoplastic effect was seen. Interestingly, 50% of the pigs in the placebo group had pericarditis, compared to none in the cell-treated group, suggesting a decreased inflammatory response after MI in the cell-treated group.

Summary. MSCs grown in the G1-SCIB are viable and freely release relevant PFs; G1-SCIBs are safe and well-tolerated and provides immunoprotection for contained cells for up to 4 weeks when deployed in the superior vena cava in the pig; and SCIB-based cell therapy using allogeneic MSCs favorably reduced adverse remodeling 4 weeks after MI in pigs.

### EXAMPLE 2

In Vitro and In Vivo Testing of Second Generation (G2) Stem Cell Implantable Bioreactor (SCIB) with Enhanced Paracrine Factor Permeability and Inner Hydrogel Matrix.

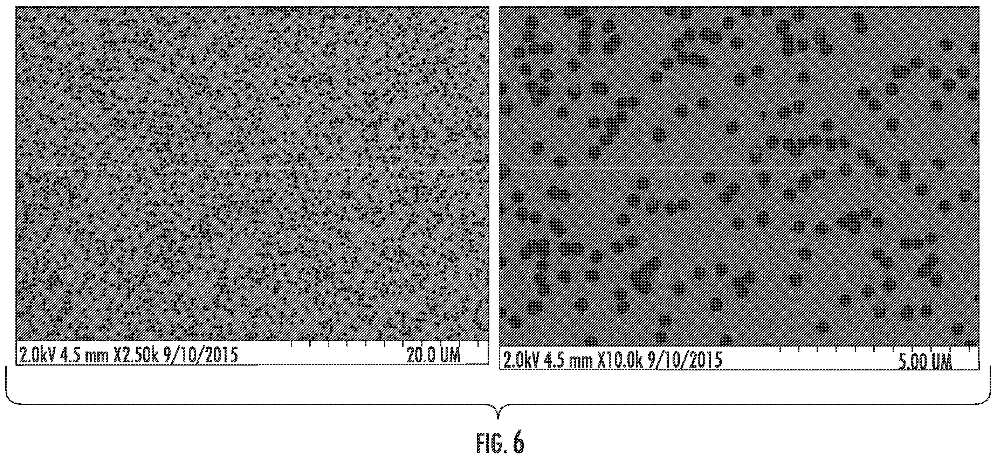

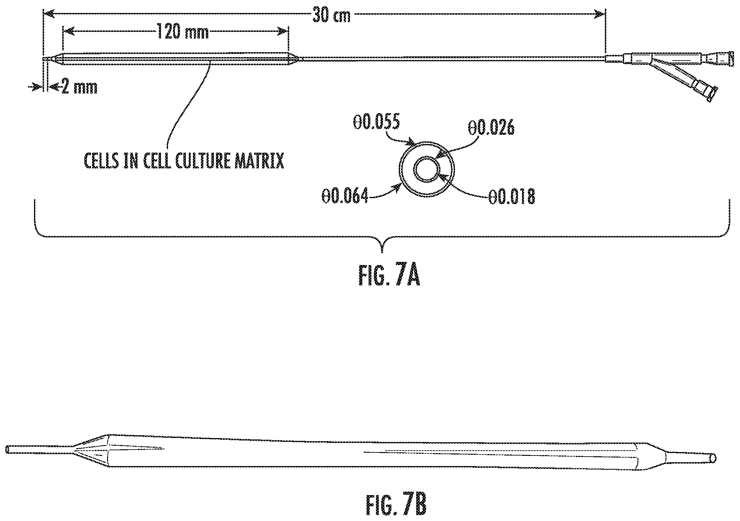

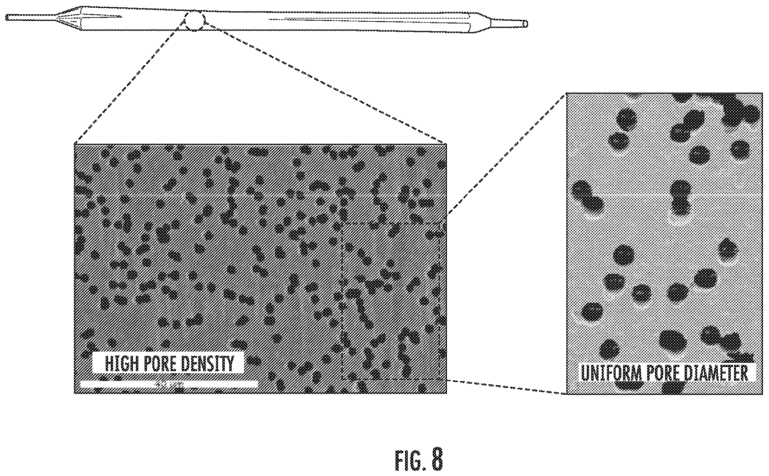

Prototypes of a second-generation SCIB (G2-SCIB) were constructed with a stem cell pouch based on a 25-µm thick polyethylene terephthalate (PET) film and incorporating a highly porous hyaluronan hydrogel to encapsulate mesenchymal stem cells within the pouch (Figs. 7A-7B). Pores in the PET film were created using track-etching, a technique that allows the creation of a dense field of highly uniform circular pores (Figs. 6, 8). The PET film is first irradiated by mono-energetic heavy ions accelerated by a cyclotron under high vacuum to create linear damage tracks through the film. The damage tracks in the irradiated film are then processed in successive alkaline hydrolysis baths before acid neutralization and washings with demineralized water, etching uniform cylindrical pores with pore size and density that can be tightly regulated by variation of irradiation time, etch base and acid concentration, etch temperature, and etch duration. Stem cell pouches of the prototype G2-SCIBs were created with pore diameters ranging from 0.44 µm to 2.0 µm at a density ranging from 2.4 million pores per square cm to 8.0 million pores per square cm, but as detailed above, smaller or larger pore diameters as well as lower or higher pore densities are feasible. Since exosomes are generally less than 0.2 µm in diameter and cells are generally more than 8 µm in diameter, the pores in the G2-SCIB allow the pouch to be completely permeable to the exosomal fraction of PFs (unlike the G1-SCIB), while maintaining impermeability to cells.

Permeability of G2-SCIB to Proteins and Exosomes.

G2-SCIB pouch permeability to FITC-albumin was measured over 2 hours. Albumin, with a molecular weight of 66 kD (larger than or comparable in size to many growth factor paracrine factors), crossed the track-etched PET membrane with 0.44-µm pores into a surrounding bath without hindrance, with 20% release from the SCIB within 30 minutes, and near 100% release within less than 2 hours (Fig. 10A). Exosomes also readily cross the G2-SCIB pouch membrane. Rat H9c2 cardiomyoblasts avidly incorporated exosomes which freely migrated across the track-etched PET membrane (Fig. 10B). At the same time, no H9c2 cardiomyoblasts were seen to cross the membrane, consistent with the significantly larger cell diameter compared to the track-etched pore diameter.

Stem Cell Viability in G2-SCIB is Enhanced by Hydrogel Matrix.

Luciferase-expressing mouse MSCs were grown in 4.0 mm diameter X 32 mm long cylindrical SCIB track-etched PET pouches at a dose of 10⁶ cells for a duration of 14 days with and without HyStem HP hyaluronan hydrogel infused into the pouch. Flow cytometry using markers CD44, Sca-1, CD31, CD45, and CD34 show that the MSCs retained their cell surface marker expression profile. Viability of the MSCs was assessed using luciferase bioluminescence imaging (BLI) for up to 14 days in culture. Mouse MSCs in G2-SCIB pouches incorporating an inner hydrogel matrix maintained significantly greater viability compared to those in G2-SCIB pouches without an inner hydrogel matrix (Fig. 11A). The 4-fold increase in mouse MSC viability when a hydrogel matrix in incorporated in the pouch persisted for up to 14 days in culture (Fig. 11B).

PF Production and Tubulogenesis in G2-SCIB.

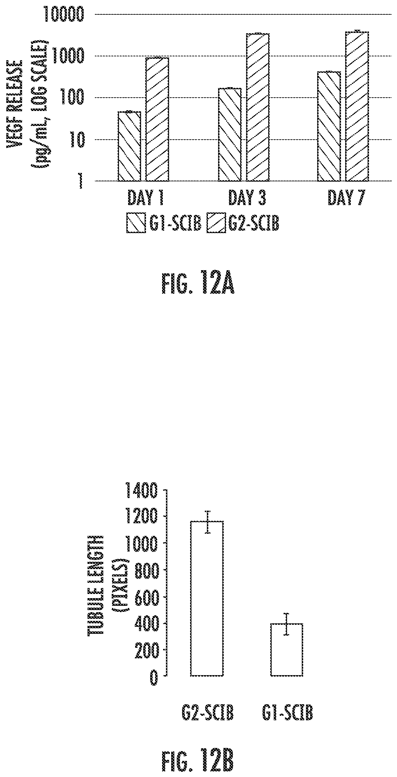

Human MSCs grown in G2-SCIBs incorporating hyaluronan hydrogel and in G1-SCIBs of similar dimensions, and relative PF production and release was assessed. Elaboration of VEGF, a representative angiogenic PF, was measured by ELISA. G2-SCIB production of VEGF was uniformly in excess of 10-fold greater than the G1-SCIB throughout the 7-day culture period (Fig. 12A). Consistent with this, *in vitro* endothelial cell tubulogenesis induced by paracrine factors released from the G2-SCIB after 8 days in culture was also significantly greater than that induced by the G1-SCIB (Fig. 12B). The improved *in vitro* tubulogenesis potential of the G2-SCIB can be accounted for by the presence of the inner hydrogel matrix. When G2-SCIB without an inner hydrogel matrix was compared to the G1-SCIB, there was no longer a significant difference in the degree of tubulogenesis.

G2-SCIB Biocompatibility and Immunoprotection.

A G2-SCIB loaded with 5 x 10⁶ mouse MSCs expressing luciferase was implanted in the superior vena cava of a pig for two weeks; control G2-SCIBs loaded with the same number of cells were kept in culture. Despite the use of xenogeneic cells, the animal tolerated the implantation well, without any evidence of immune reaction, transfusion reaction, or thrombosis. After explantation, bioluminescence imaging demonstrated 35% greater MSC viability in the implanted G2-SCIB as compared to the *in vitro* G2-SCIB kept in culture. These data demonstrated that immunoprotection and biocompatibility are maintained by the G2-SCIB; moreover, the greater MSC viability in the implanted G2-SCIB implies that the efficiency of transfer of nutrients and wastes across the SCIB stem cell pouch membrane is improved by circulating blood flow.

G2-SCIB Cell Viability with Microbeads

MSCs were loaded onto polystyrene microbeads (106 µm to 125 µm diameter were used, but larger or smaller diameters are also feasible). The microbeads were sterilized then coated with fibronectin and seeded with MSCs. The MSCs were cultured on the microbeads for 3 days. The MSC-coated microbeads were then infused into the cell chamber of the SCIB at a density of approximately 600,000 to 1.2 million microbeads per 3.4 ml of cell chamber volume. Higher or lower microbead densities can also be used to achieve different cell loading levels. After 7 days in the cell chamber, MSCs remained attached to the microbeads, and cell viability on the microbeads after 7 days of culture in the cell pouch exceeded 90% (Fig. 9) by the live/green dead/red fluorescence viability assay.

### Summary.

Testing of a G2-SCIB with a novel track-etched PET pouch incorporating an inner hyaluronan hydrogel matrix demonstrated permeability to large proteins and to exosomes, impermeability to cells, and fostered excellent stem cell viability with high levels of PF production and endothelial cell tubulogenesis, while maintaining *in vivo* biocompatibility and immunoprotection.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The use of any and all examples, or exemplary language (e.g., "such as") provided herein, is intended merely to better illuminate the invention and does not pose a limitation on the scope of the invention unless otherwise claimed.

Preferred embodiments of this invention are described herein, including the best mode known to the inventors for carrying out the invention.

## Claims

1. An implantable bioreactor, comprising:
a) one or more enclosures defining an enclosed space with an interior and exterior surface, the one or more enclosures comprise at least one semi-permeable layer of material located in between and in contact or adjacent to at least a first and second permeable layer of material, wherein the first permeable layer of material is on the exterior facing surface of the enclosure, and the second layer of permeable material is on the interior facing surface of the enclosure, the one or more enclosures being semi-permeable such that it can provide containment of cells in the enclosed space and prevent the egress of the cells while also providing an immunological barrier, and the one or more enclosures being capable of allowing egress of paracrine factors out of the enclosure, wherein said enclosure has pores with diameters in a range of 50 nm to 5,000 nm; and
b) cells within a cell culture matrix within the one or more enclosures, said cells being capable of producing paracrine factors and said cell culture matrix comprising a hydrogel.

2. An implantable bioreactor in accordance with claim 1, wherein the one or more enclosures comprise the at least one semi-permeable layer of material in contact or adjacent to at least one layer of cells within a cell culture matrix, and the layer of cells is on the interior facing surface of the one or more enclosures, and is capable of being rolled or folded.

3. The implantable bioreactor of claim 1 or claim 2, wherein the one or more enclosures are collapsible or expandable or both.

4. The implantable bioreactor of any of claims 1 to 3, wherein said enclosure is comprised of biologic and/or synthetic materials.

5. The implantable bioreactor of claim 4, wherein said biologic material is cellulose acetate.

6. The implantable bioreactor of claim 4, wherein said synthetic material is selected from the group consisting of polysulfone, polyamide, polacrylonitrile, copolymers thereof, polyethylene terephthalate (PET), polymethylmetacrylate, polytetrafluroethylene and derivatives thereof, polycarbonates and derivatives, poly(ethylene-co-vinyl acetate) and derivatives, poly(n-butyl methacrylate) and derivatives, poly(styrene-b-isobutylene-b-styrene) and derivatives, polycaprolactone and derivatives, polyimides and derivatives, polyurethanes and derivatives, poly(lactic acid), poly(lactide-co-glycolide), and silicones.

7. The implantable bioreactor of any of claims 1 to 3, wherein said enclosure has pore densities ranging from 100,000 to 100 million pores per square centimeter.

8. The implantable bioreactor of any of claims 1 to 7, wherein said cells are stem cells.

9. The implantable bioreactor of claim 8, wherein the stem cells are selected from the group consisting of embryonic stem cells, mesenchymal stem cells, adipose-derived stem cells and endothelial stem cells.

10. The implantable bioreactor of any of claims 1 to 9, wherein the cell culture matrix includes one or more compositions selected from the group consisting of cell culture media, microbeads, hydrogel (e.g., hyaluronan-based, PEG-based, or collagen-based hydrogels), polymer matrix, tissue engineering scaffold, nanofiber (e.g., PLGA-based nanofiber), and biological extracellular matrix.

11. The implantable bioreactor of any of claims 1 to 3, wherein the bioreactor enclosure is mounted on a wire, a catheter or as part of another implantable medical device.

12. The implantable bioreactor of any of claims 1 to 7, wherein said cells comprise stem cells and supporting cells.

13. The implantable bioreactor of any of claims 1 to 3, wherein said enclosure defines a port configured to be accessible to permit a containment space defined by said enclosure to be at least one of emptied, filled or refilled.

14. The implantable bioreactor of claim 13, further comprising a catheter having a distal end and a proximal end, said distal end of said catheter being attached to said enclosure through said port,
wherein said catheter is at least partially implantable such that said distal end of said catheter is implantable while said proximal end of said catheter is adapted to extend exterior to a patient's body while in use.

## Patentansprüche

1. Ein implantierbarer Bioreaktor, der Folgendes beinhaltet:
a) ein oder mehrere Gehäuse, die einen umschlossenen Raum mit einer inneren und äußeren Oberfläche definieren, wobei das eine oder die mehreren Gehäuse mindestens eine halbdurchlässige Materialschicht beinhalten, die zwischen und in Kontakt mit oder angrenzend an mindestens eine erste und zweite durchlässige Materialschicht angeordnet ist, wobei sich die erste durchlässige Materialschicht auf der nach außen weisenden Oberfläche des Gehäuses befindet und sich die zweite Schicht aus durchlässigem Material auf der nach innen weisenden Oberfläche des Gehäuses befindet, wobei das eine oder die mehreren Gehäuse halbdurchlässig sind, sodass sie ein Einschließen von Zellen in dem umschlossenen Raum bereitstellen und den Austritt der Zellen verhindern können, während sie auch eine immunologische Barriere bereitstellen, und wobei das eine oder die mehreren Gehäuse in der Lage sind, den Austritt von parakrinen Faktoren aus dem Gehäuse zu ermöglichen, wobei das Gehäuse Poren mit Durchmessern in einem Bereich von 50 nm bis 5 000 nm aufweist; und
b) Zellen innerhalb einer Zellkulturmatrix innerhalb des einen oder der mehreren Gehäuse, wobei die Zellen in der Lage sind, parakrine Faktoren zu produzieren, und wobei die Zellkulturmatrix ein Hydrogel beinhaltet.

2. Implantierbarer Bioreaktor gemäß Anspruch 1, wobei das eine oder die mehreren Gehäuse die mindestens eine halbdurchlässige Materialschicht in Kontakt mit oder angrenzend an mindestens eine Zellschicht innerhalb einer Zellkulturmatrix beinhalten und sich die Zellschicht auf der nach innen weisenden Oberfläche des einen oder der mehreren Gehäuse befindet und in der Lage ist, gerollt oder gefaltet zu werden.

3. Implantierbarer Bioreaktor gemäß Anspruch 1 oder Anspruch 2, wobei das eine oder die mehreren Gehäuse zusammenklappbar oder erweiterbar oder beides sind.

4. Implantierbarer Bioreaktor gemäß einem der Ansprüche 1 bis 3, wobei das Gehäuse aus biologischem und/oder synthetischem Material besteht.

5. Implantierbarer Bioreaktor gemäß Anspruch 4, wobei das biologische Material Celluloseacetat ist.

6. Implantierbarer Bioreaktor gemäß Anspruch 4, wobei das synthetische Material aus der Gruppe ausgewählt ist, die aus Polysulfon, Polyamid, Polyacrylnitril, Copolymeren davon, Polyethylenterephthalat (PET), Polymethylmetacrylat, Polytetrafluorethylen und Derivaten davon, Polycarbonaten und Derivaten, Poly(ethylen-co-vinylacetat) und Derivaten, Poly(n-butylmethacrylat) und Derivaten, Poly(styrol-b-isobutylen-b-styrol) und Derivaten, Polycaprolacton und Derivaten, Polyimiden und Derivaten, Polyurethanen und Derivaten, Poly(milchsäure), Poly(lactid-co-glycolid) und Silikonen besteht.

7. Implantierbarer Bioreaktor gemäß einem der Ansprüche 1 bis 3, wobei das Gehäuse Porendichten im Bereich von 100 000 bis 100 Millionen Poren pro Quadratzentimeter aufweist.

8. Implantierbarer Bioreaktor gemäß einem der Ansprüche 1 bis 7, wobei die Zellen Stammzellen sind.

9. Implantierbarer Bioreaktor gemäß Anspruch 8, wobei die Stammzellen aus der Gruppe ausgewählt sind, die aus embryonalen Stammzellen, mesenchymalen Stammzellen, aus Fettgewebe stammenden Stammzellen und endothelialen Stammzellen besteht.

10. Implantierbarer Bioreaktor gemäß einem der Ansprüche 1 bis 9, wobei die Zellkulturmatrix eine oder mehrere Zusammensetzungen umfasst, die aus der Gruppe ausgewählt sind, die aus Zellkulturmedien, Mikrokügelchen, Hydrogel (z. B. Hydrogelen auf Hyaluronanbasis, PEG-Basis oder Kollagenbasis), Polymermatrix, Gewebezüchtungsgerüst, Nanofaser (z. B. Nanofaser auf PLGA-Basis) und biologischer extrazellulärer Matrix besteht.

11. Implantierbarer Bioreaktor gemäß einem der Ansprüche 1 bis 3, wobei das Bioreaktorgehäuse an einem Draht, einem Katheter oder als Teil einer anderen implantierbaren medizinischen Vorrichtung montiert ist.

12. Implantierbarer Bioreaktor gemäß einem der Ansprüche 1 bis 7, wobei die Zellen Stammzellen und Stützzellen beinhalten.

13. Implantierbarer Bioreaktor gemäß einem der Ansprüche 1 bis 3, wobei das Gehäuse eine Öffnung definiert, die konfiguriert ist, um zugänglich zu sein, um zu gestatten, dass ein durch das Gehäuse definierter Einschlussraum mindestens eines von entleert, gefüllt oder nachgefüllt wird.

14. Implantierbarer Bioreaktor gemäß Anspruch 13, der ferner einen Katheter beinhaltet, der ein distales Ende und ein proximales Ende aufweist, wobei das distale Ende des Katheters durch die Öffnung an dem Gehäuse angebracht ist,
wobei der Katheter mindestens teilweise implantierbar ist, sodass das distale Ende des Katheters implantierbar ist, während das proximale Ende des Katheters angepasst ist, um sich im Gebrauch außerhalb des Körpers eines Patienten zu erstrecken.

## Revendications

1. Un bioréacteur implantable, comprenant :
a) une ou plusieurs enceintes définissant un espace clos avec une surface intérieure et une surface extérieure, les une ou plusieurs enceintes comprenant au moins une couche semi-perméable de matériau située entre et en contact avec ou adjacente à au moins une première et une deuxième couche perméable de matériau, dans lequel la première couche perméable de matériau est sur la surface tournée vers l'extérieur de l'enceinte, et la deuxième couche de matériau perméable est sur la surface tournée vers l'intérieur de l'enceinte, les une ou plusieurs enceintes étant semi-perméables de sorte qu'elles peuvent assurer le confinement de cellules dans l'espace clos et empêcher les cellules de sortir tout en assurant également une barrière immunologique, et les une ou plusieurs enceintes étant capables de laisser sortir des facteurs paracrines hors de l'enceinte, dans lequel ladite enceinte a des pores dont les diamètres sont compris dans une plage de 50 nm à 5 000 nm ; et
b) des cellules à l'intérieur d'une matrice de culture cellulaire à l'intérieur des une ou plusieurs enceintes, lesdites cellules étant capables de produire des facteurs paracrines et ladite matrice de culture cellulaire comprenant un hydrogel.

2. Un bioréacteur implantable selon la revendication 1, dans lequel les une ou plusieurs enceintes comprennent l'au moins une couche semi-perméable de matériau en contact avec ou adjacente à au moins une couche de cellules à l'intérieur d'une matrice de culture cellulaire, et la couche de cellules est sur la surface tournée vers l'intérieur des une ou plusieurs enceintes, et est capable d'être enroulée ou pliée.

3. Le bioréacteur implantable de la revendication 1 ou de la revendication 2, dans lequel les une ou plusieurs enceintes sont compressibles ou expansibles ou les deux.

4. Le bioréacteur implantable de n'importe lesquelles des revendications 1 à 3, dans lequel ladite enceinte est constituée de matériaux biologiques et/ou synthétiques.

5. Le bioréacteur implantable de la revendication 4, dans lequel ledit matériau biologique est de l'acétate de cellulose.

6. Le bioréacteur implantable de la revendication 4, dans lequel ledit matériau synthétique est sélectionné dans le groupe constitué de polysulfone, de polyamide, de polyacrylonitrile, de copolymères de ceux-ci, de polyéthylène téréphtalate (PET), de polyméthylméthacrylate, de polytétrafluoroéthylène et de dérivés de ceux-ci, de polycarbonates et dérivés, de poly(éthylène-co-acétate de vinyle) et dérivés, de poly(méthacrylate de n-butyle) et dérivés, de poly(styrène-b-isobutylène-b-styrène) et dérivés, de polycaprolactone et dérivés, de polyimides et dérivés, de polyuréthanes et dérivés, de poly(acide lactique), de poly(lactide-co-glycolide), et de silicones.

7. Le bioréacteur implantable de n'importe lesquelles des revendications 1 à 3, dans lequel ladite enceinte a des densités de pores allant de 100 000 à 100 millions de pores par centimètre carré.

8. Le bioréacteur implantable de n'importe lesquelles des revendications 1 à 7, dans lequel lesdites cellules sont des cellules souches.

9. Le bioréacteur implantable de la revendication 8, dans lequel les cellules souches sont sélectionnées dans le groupe constitué de cellules souches embryonnaires, de cellules souches mésenchymateuses, de cellules souches dérivées de tissu adipeux et de cellules souches endothéliales.

10. Le bioréacteur implantable de n'importe lesquelles des revendications 1 à 9, dans lequel la matrice de culture cellulaire inclut une ou plusieurs compositions sélectionnées dans le groupe constitué de milieux de culture cellulaire, de microbilles, d'hydrogel (par exemple, des hydrogels à base de hyaluronane, à base de PEG, ou à base de collagène), de matrice polymère, d'échafaudage d'ingénierie tissulaire, de nanofibre (par exemple, de nanofibre à base de PLGA), et de matrice extracellulaire biologique.

11. Le bioréacteur implantable de n'importe lesquelles des revendications 1 à 3, dans lequel l'enceinte de bioréacteur est montée sur un fil, un cathéter ou en tant que partie d'un autre dispositif médical implantable.

12. Le bioréacteur implantable de n'importe lesquelles des revendications 1 à 7, dans lequel lesdites cellules comprennent des cellules souches et des cellules supportrices.

13. Le bioréacteur implantable de n'importe lesquelles des revendications 1 à 3, dans lequel ladite enceinte définit un orifice configuré pour être accessible afin de permettre à un espace de confinement défini par ladite enceinte d'être au moins soit vidé, soit rempli, soit rerempli.

14. Le bioréacteur implantable de la revendication 13, comprenant en outre un cathéter ayant une extrémité distale et une extrémité proximale, ladite extrémité distale dudit cathéter étant attachée à ladite enceinte à travers ledit orifice,
dans lequel ledit cathéter est au moins partiellement implantable de telle sorte que ladite extrémité distale dudit cathéter est implantable tandis que ladite extrémité proximale dudit cathéter est adaptée pour s'étendre à l'extérieur du corps d'un patient lors de l'utilisation.
